# EUROPEAN PATENT SPECIFICATION

(11) **EP 2 331 957 B1**
(45) Date of publication and mention of the grant of the patent: **02.11.2016**
(21) Application number: 09737160.3
(22) Date of filing: 01.09.2009
(51) Int. Cl.: G01N 33/50

(54) **METHODS OF SELECTION OF CELLS FOR TRANSPLANTATION**
AUSWAHLVERFAHREN FÜR ZELLEN ZUR TRANSPLANTATION
MÉTHODES DE SÉLECTION DE CELLULES POUR UNE TRANSPLANTATION

(30) Priority: 02.09.2008 US 136374 P
(43) Date of publication of application: 15.06.2011
(73) Proprietor: Pluristem Ltd., 31905 Haifa (IL)
(72) Inventor: MEIRON, Moran, 30900 Zikhron-Yaakov (IL); TOREN, Amir, 30900 Zikhron-Yaakov (IL); OFIR, Rachel, 17940 Mitzpe Adi (IL)
(74) Representative: Dörries, Hans Ulrich
(86) International application number: PCT/IL2009/000844
(87) International publication number: WO 2010/026573

(56) References cited:
- EP-A1- 1 845 154
- WO-A2-02/064755
- WO-A2-2007/108003
- ZHOU B ET AL: "Therapeutic neovascularization for peripheral arterial diseases: advances and perspectives" HISTOLOGY AND HISTOPATHOLOGY, MURCIA, ES, vol. 22, no. 6, 1 June 2007 (2007-06-01), pages 677-686, XP009127650 ISSN: 0213-3911

## Description

### FIELD AND BACKGROUND OF THE INVENTION

The present invention, in some embodiments thereof, relates to adherent cells of a placenta tissue and, more particularly, but not exclusively, to methods of selection of same for transplantation.

Peripheral arterial disease (PAD), also known as Peripheral Vascular Disease (PVD), occurs when peripheral arteries are damaged by arterial hypertension and/or by the formation of atherosclerotic plaques. Approximately 8 million patients in the US suffer from PAD. PAD is a chronic disease that progressively constricts arterial circulation of limbs that can lead to serious medical complications. This disease is often associated with other clinical conditions, including hypertension, cardiovascular disease, hyperlipidemia, diabetes, obesity and stroke.

Critical Limb Ischemia (CLI) is used to describe patients with chronic ischemia induced pain, ulcers, tissue loss or gangrene in the limb. CLI is usually associated with smoking, hyperlipidemia, hypertension, diabetes, hyperhomocysteinemia and has hereditary susceptibility (i.e., strong family history of similar disease). Typically, CLI symptoms include limb pain at rest with or without trophic skin changes or tissue loss, pain is typically worse when a patient is supine, pain typically improves when limb is in the dependent position. Furthermore, CLI is usually associated with ulceration or tissue loss and gangrene of the extremity. Also, typically, narcotic medications are required for analgesia.

CLI represents the end stage of PAD patients who need comprehensive treatment by a vascular surgery or vascular specialist. However, in contrast to coronary and cerebral artery disease, peripheral arterial disease (PAD) remains an under-appreciated condition that despite being serious and extremely prevalent is rarely diagnosed and even less frequently treated. Consequently, CLI often leads to amputation or death and mortality rates in PAD patients exceed that of patients with myocardial infarction and stroke.

Conventional treatments for CLI include surgery, including e.g. primary amputation, medical management (e.g. clopidogrel, cilostazol, statins) and lifestyle modifications (e.g. cessation of smoking). Furthermore, in an attempt to treat ischemic conditions, stem cell therapy has been contemplated with various adult stem cells [Nakagami et al., J Atheroscler Thromb (2006) 13(2): 77-81; Moon et al.,Cell Physiol Biochem. (2006) 17: 279-90; Iwase el al., Cardiovasc Res. (2005) 66(3):543-51; Ventura et al., (2007) J. Biol. Chem., 282: 14243-52].

The traditional source of mesenchymal stem cells for therapeutic use has been bone marrow. More recently novel sources of MSC are being investigated for potential clinical use for their regenerative potential and immunomodulatory function. To obtain sufficient numbers of MSC for therapeutic use, ex vivo expansion is necessary. This requires optimization of culture conditions and development of defined media, animal (xeno) product-free or low risk sources or components including alternatives to fetal calf serum, trypsin and other reagents. Furthermore a clear definition of release criteria of transplanted cells to be used as a clinical therapy is not yet uniformly accepted.

Bone marrow harvest is a routine procedure and therefore whole bone marrow or bone marrow MSC are most commonly used in MSC clinical therapies. However, the collection of bone marrow is an invasive procedure. Other sources of MSC have been demonstrated including liver, perdiodontal ligaments, adipose tissue, placenta (WO/2007/108003). The ideal source of MSC for the clinic would be readily available, using a non-invasive harvesting procedure and yielding large numbers of MSC for ex vivo expansion. In this respect placenta represents a particularly attractive source due to ready availability.

Currently, most clinical production of MSC involves ex vivo expansion culture in media containing xeno contaminants. This raised the potential problem of prion and viral transmission and immune response to xenogeneic antigens. Recommendations for release criteria have not been standardized for MSC from any source.

### SUMMARY OF THE INVENTION

According to an aspect of some embodiments of the present invention there is provided a method of selecting a population of adherent cells of a placenta tissue suitable for treating ischemia, the method comprising: (a) determining prior to transplantation in a candidate population of adherent cells of a placenta tissue at least one of the following parameters: (i) percentage of viable cells in the candidate population; (ii) immune phenotype of cells in the candidate population; (iii) xeno-contamination in the candidate population; (iv) sterility of the candidate population; and (v) immunosuppressive activity of cells in the candidate population; (b) selecting the candidate population according to predetermined values of at least one of the parameters, thereby selecting a population of adherent cells of the placenta tissue suitable for treating ischemia.

According to some embodiments of the invention, the percentage of viable cells is at least 70 %.

According to some embodiments of the invention, the immune phenotype comprises a positive marker expression of CD73, CD29 and CD105 and a negative marker expression of of CD45, CD14 and HLA-DR.

According to some embodiments of the invention, the cells comprising the positive marker expression make up at least 90 % of the candidate population and cells comprising the negative marker expression make up equally to- or less than 3 % of the candidate population.

According to some embodiments of the invention, the xeno-contamination is selected from the group consisting of mycoplasma contamination and endotoxin contamination.

According to some embodiments of the invention, the selecting is determined according to the values of all of the parameters.

According to some embodiments of the invention, the selecting is according to the following values: (i) at least 70 % of viable cells in the candidate population; and (ii) immune phenotype comprising a positive marker expression of CD73, CD90 and CD105 and a negative marker expression of CD45, CD14 and HLA-DR of cells in the candidate population, wherein cells comprising the positive marker expression make up at least 90 % of the candidate population and cells comprising the negative marker expression make up equally to- or less than 3 % of the candidate population.

According to some embodiments of the invention, the selecting further comprising at least one of: (iii) no xeno-contamination in the candidate population; (iv) sterility of the candidate population; and (v) immunosuppressive activity of cells in the candidate population;

According to some embodiments of the invention, the adherent cells are ex-vivo expanded.

According to some embodiments of the invention, the adherent cells are ex vivo expanded under 3D culturing conditions.

According to some embodiments of the invention, the 3D culturing conditions is effected under perfusion.

According to an aspect of some embodiments of the present invention there is provided a method of treating peripheral artery disease (PAD) in a subject in need thereof, the method comprising administering to the subject a therapeutically effective amount of the population of adherent cells of a placenta tissue selected suitable for transplantation according to any of the above methods.

According to an aspect of some embodiments of the present invention there is provided a use of the population of cells for the manufacture of a medicament identified for transplantation.

According to an aspect of some embodiments of the present invention there is provided a population of cells selected according to the above method.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

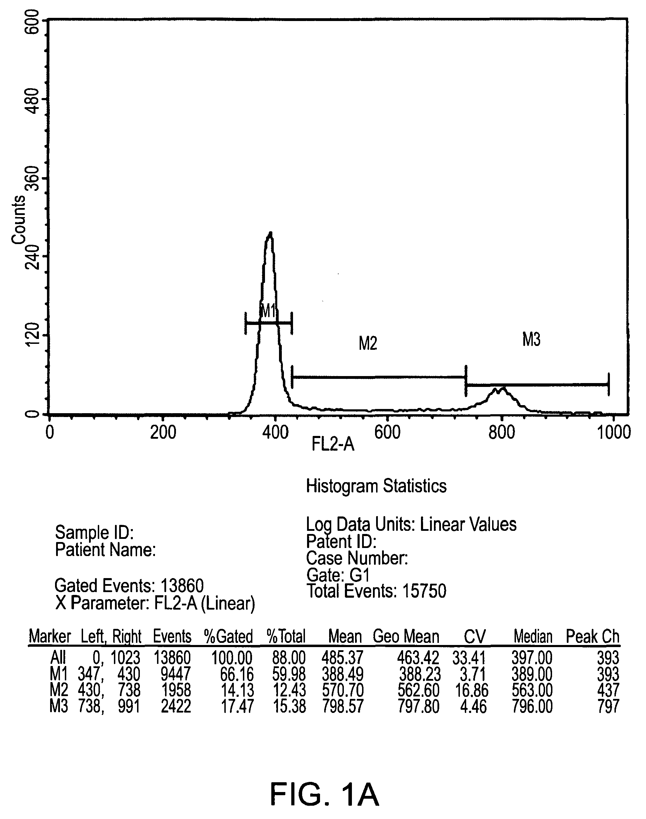

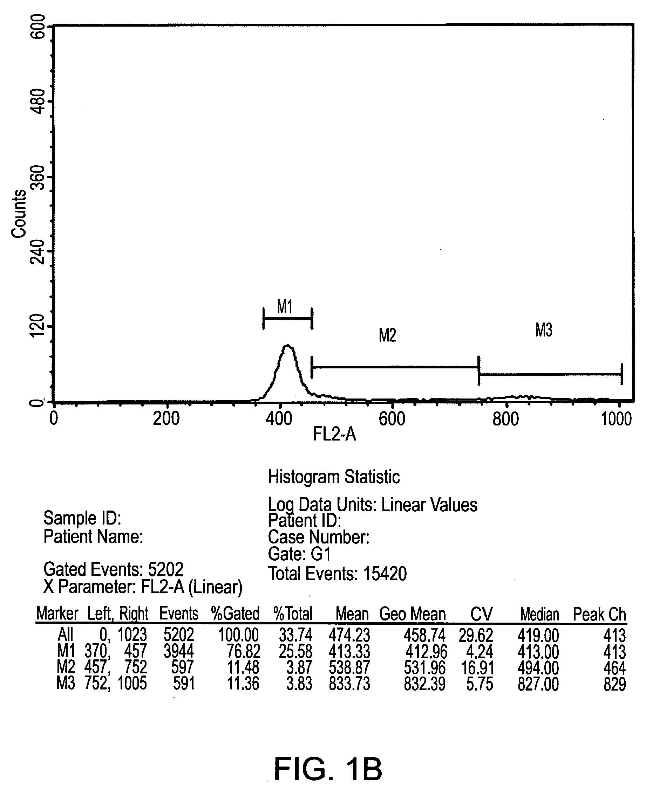

FIGs. 1A-B depict cell cycle analysis of 3D adherent cells manufactured by Plurix as was previously described in WO/2007/108003 (designated PLX, Figure 1B) and by Celligen - the teachings of the present invention (designated PLX-C, Figure 1A). Cells were fixed in 70 % EtOH O.N, centrifuged and re-suspended in a Propidium Iodide (PI) solution and then analyzed by FACS.

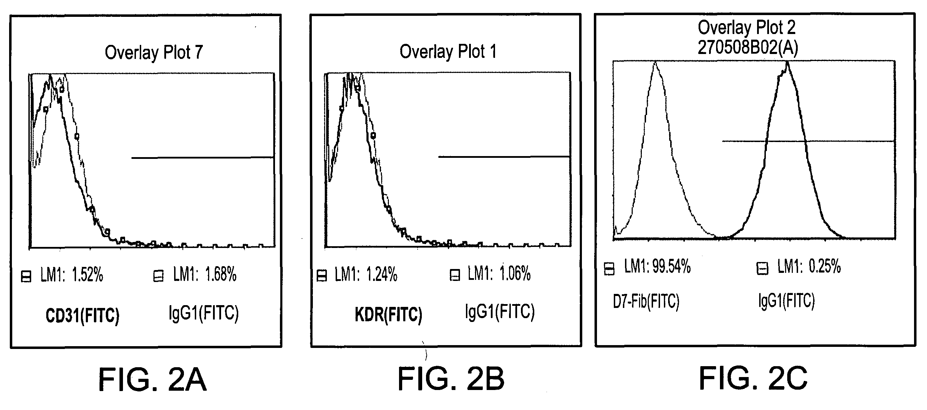

FIGs. 2A-C depict expression of fibroblast-typical markers but not expression of endothelial typical markers on PLX-C. Figure 2A depicts negative expression of the endothelial marker CD31; Figure 2B depicts negative expression of the endothelial marker KDR; and Figure 2C depicts positive expression of the human fibroblast marker (D7-FIB). Of note, the red histograms for Isotype IgG1 (FITC) represent the negative control while the blue histograms represent the positively stained cells

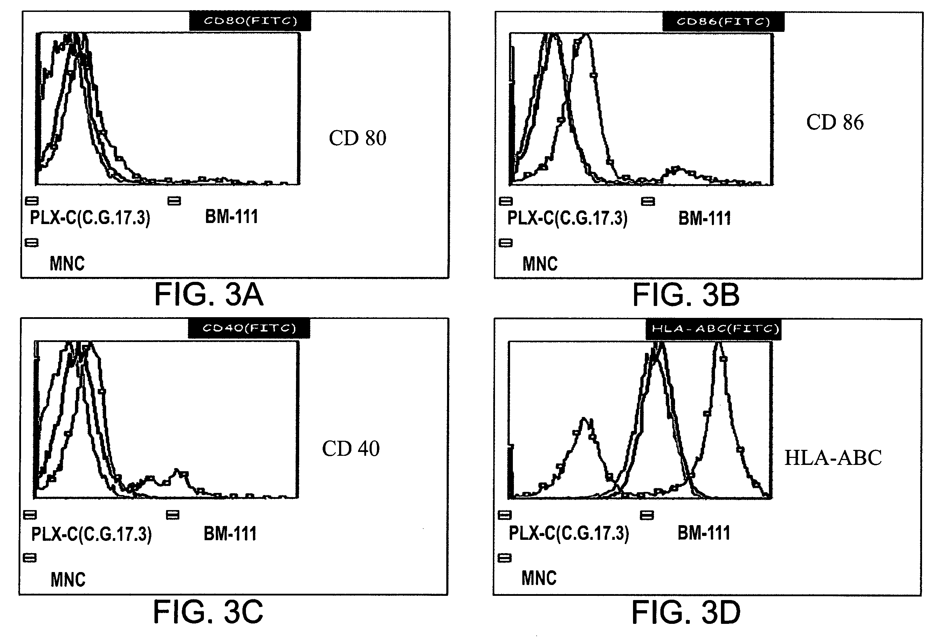

FIGs. 3A-D depict expression of stimulatory and co-stimulatory molecules on PLX-C cells. Figure 3A depicts PLX-C expression of CD80; Figure 3B depicts PLX-C expression of CD86; Figure 3C depicts PLX-C expression of CD40; and Figure 3D depicts PLX-C expression of HLA-A/B/C. Negative controls were prepared with relevant isotype fluorescence molecules. Of note, red histograms indicate PLX-C marker-expressing population of cells, blue histograms indicate bone marrow (BM) marker-expressing population of cells, and green histograms indicate mononuclear cell (MNC) marker expressing population of cells.

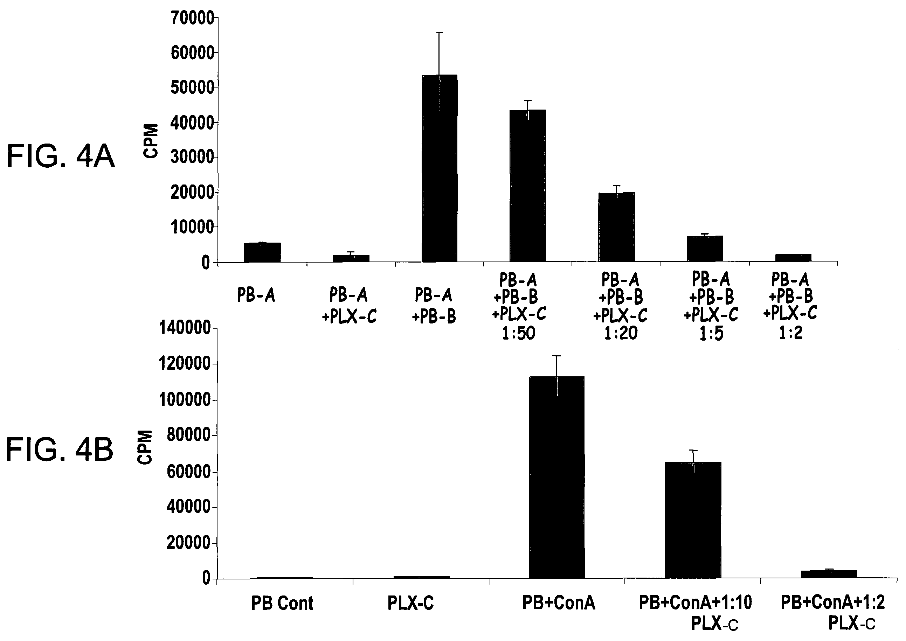

FIGs. 4A-B depict reduction of lymphocyte cell response by PLX-C. Figure 4A depicts MLR tests performed with 2 x 10⁵ peripheral blood (PB) derived MNC (donor A) stimulated with equal amount of irradiated (3000 Rad) PB derived MNCs (donor B) followed by addition of increasing amounts of PLX-C cells to the cultures; Figure 4B depict peripheral blood (PB) derived MNCs stimulated with ConA (1.5 mg/ml). Increasing amounts of PLX-C cells were added to the cultures. Three replicates of each group were seeded in 96-well plates.

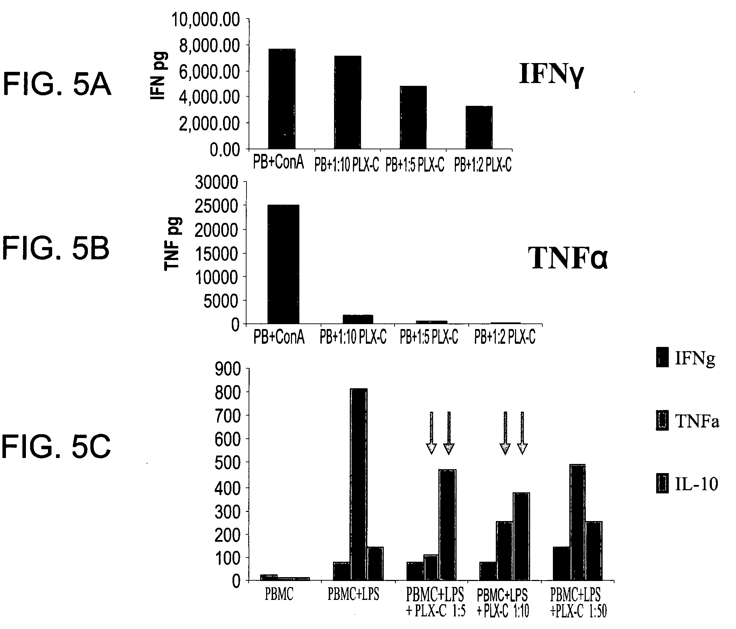

FIGs 5A-C depict PLX-C regulation of pro-inflammatory and anti-inflammatory cytokine secretion following co-culture with peripheral blood cells. Figures 5A-B depict secretion of IFNγ (Figure 5A) and TNFα (Figure 5B) following co-culture of human derived MNCs (isolated from peripheral blood) stimulated with ConA with PLX-C; Figure 5C depicts secretion of IFNγ, TNFα and IL-10 following co-culture of human derived MNCs (isolated from peripheral blood) stimulated with LPS with PLX-C. Supernatants were collected and subjected to cytokines analysis using ELISA.

### DESCRIPTION OF SPECIFIC EMBODIMENTS OF THE INVENTION

The present invention, in some embodiments thereof, relates to adherent cells of a placenta tissue and, more particularly, but not exclusively, to methods of selection of same for transplantation.

The principles and operation of the present invention may be better understood with reference to the drawings and accompanying descriptions.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Embodiments of the present invention provide criteria for selection of populations of adherent cells from a placenta tissue with a high probability of effective engraftment and therapeutic efficacy. The selected populations can be used for transplantation in the clinical setting such as for treatment of a variety of medical conditions, including peripheral artery disease (PAD).

Thus, according to one aspect of the present invention, there is provided a method of selecting a population of adherent cells of a placenta tissue suitable for treating ischemia, the method comprising:
(a) determining prior to transplantation in a candidate population of adherent cells of a placenta tissue at least one of the following parameters:
   (i) percentage of viable cells in the candidate population;
   (ii) immune phenotype of cells in the candidate population;
   (iii) xeno-contamination in the candidate population;
   (iv) sterility of the candidate population; and
   (v) immunosuppressive activity of cells in the candidate population;
(b) selecting the candidate population according to predetermined values of at least one of the parameters, thereby selecting a population of adherent cells of the placenta tissue suitable for treating ischemia.

As used herein the phrase "population of cells" refers to a homogeneous or heterogeneous isolated population of cells which comprise cell populations potentially suitable for transplantation. The candidate population of cells in accordance with the present teachings comprises adherent cells of a placenta tissue.

As used herein the phrase "adherent cells" refers to a population of cells which are anchorage dependent, i.e., require attachment to a surface in order to grow *in vitro.*

As used herein the term "placenta tissue" refers to any portion of the mammalian organ which lines the uterine wall and during pregnancy envelopes the fetus, to which it is attached by the umbilical cord. Following birth, the placenta is expelled (and is referred to as a post partum placenta). In an exemplary embodiment, placenta refers to whole placenta.

As used herein, the term "viability" or "viable" refers to the distinction between living and non-living cells. Cell viability may be judged by morphological changes or by changes in membrane permeability and/or physiological state inferred from the exclusion of certain dyes or the uptake and retention of others. Cell viability assays are well known in the art, including, but not limited to trypan blue or propidium iodide exclusion and rhodamine metabolic stain (Coder, D., Current Protocols in Cytometry, 1997, John Wiley and Sons, Inc., Unit 9.2, 9.2.1-9.2.14). According to a specific embodiment of the present invention, the percentage of viable cells in the population is at least about 70 %, 80 %, 90 % or more.

As used herein the phrase "immune phenotype" refers to an expression profile of cell surface markers. In a specific embodiment the immune phenotype comprises a positive marker expression of CD73, CD90 and CD105 and a negative marker expression of CD45, CD14 and HLA-DR. Marker expression can be determined at the protein or mRNA level using methods which are well known in the art and are further described hereinbelow. According to a specific embodiment of the present invention, cells comprising positive marker expression make up at least 90 %, 95 %, 98 % or 100 % of the candidate population and cells comprising negative marker expression make up equally to- or less than, 3 %, 1 %, 0.5 % or less of the candidate population.

As used herein the phrase "xeno-contamination" refers to a substance of a biological source which is foreign to the transplanted subject (xenogeneic) and may elicit an immune response in the transplanted subject. Examples of xeno contaminants include a mycoplasma infection and presence of endotoxin. Methods of determining presence of same are well known in the art and are further described in Examples 1-2 of the Examples section which follows.

As used herein "sterility" refers to absence of infectious microorganisms. Sterility may be determined using a variety of methods known in the art which are further described in Example 2 of the Examples section which follows.

As used herein the phrase "immunosuppressive activity" refers to decreasing or inhibiting the immune reaction occurring in a subject in response to an antigen (e.g., a foreign cell or a portion thereof). The immune response which can be suppressed by the adherent cells include the humoral immune responses and cellular immune responses, which involve specific recognition of pathogen antigens via antibodies and T-lymphocytes (proliferation of T cells), respectively.

Immunosuppressive activity can be determined in a variety of methods such as using the Mixed Lymphocyte Reaction (MLR) as explained in detail in Example 1of the Examples section which follows.

According to some embodiments of the present invention, the candidate population of cells is ex vivo propagated.

As used herein the term *"ex-vivo"* refers to a process in which cells are removed from a living organism and are propagated outside the organism (e.g., in a test tube, in a cell culture bag, etc).

Placenta derived adherent cells can be propagated using two dimensional or three dimensional culturing conditions.

Conditions for propagating adherent cells in 2D culture are further described hereinbelow and in the examples section which follows.

As used herein the phrase "three dimensional culture" refers to a culture in which the cells are disposed to conditions which are compatible with cell growth including a scaffold which allows cell to cell contacts in three dimensions. It is well appreciated that the *in situ* environment of a cell in a living organism (or a tissue) is in a three dimensional architecture. Cells are surrounded by other cells. They are held in a complex network of extra cellular matrix nanoscale fibers that allows the establishment of various local microenvironments. Their extra cellular ligands mediate not only the attachment to the basal membrane but also access to a variety of vascular and lymphatic vessels. Oxygen, hormones and nutrients are ferried to cells and waste products are carried away. The conditions in the three dimensional culture of the invention are designed to mimic such an environment as is further exemplified below.

It will be appreciated that the conditions (e.g. pH, temperature etc.) of the three-dimensional culture are such that enable expansion of the adherent cells.

As used herein the terms "expanding" and "expansion" refer to substantially differentiation-less maintenance of the cells and ultimately cell growth, i.e., increase of a cell population (e.g., at least 2 fold) without differentiation accompanying such increase.

As used herein the terms "maintaining" and "maintenance" refer to substantially differentiation-less cell renewal, i.e., substantially stationary cell population without differentiation accompanying such stationarity.

As mentioned, the adherent cells of this aspect of the invention are retrieved from a placental tissue.

Placental cells may be obtained from a full-term or pre-term placenta. Placenta is preferably collected once it has been ex blooded. The placenta is preferably perfused for a period of time sufficient to remove residual cells. The term "perfuse" or "perfusion" used herein refers to the act of pouring or passaging a fluid over or through an organ or tissue. The placental tissue may be from any mammal; for example, the placental tissue is human. A convenient source of placental tissue is from a post partum placenta (e.g., 1-6 hours), however, the source of placental tissue or cells or the method of isolation of placental tissue is not critical to the invention.

Placenta derived adherent cells may be obtained from both fetal (*i.e.,* amnion, chorion, chorionic villi or inner parts of the placenta, see Example 1) and maternal (*i.e*., decidua basalis, and decidua parietalis) parts of the placenta. Tissue specimens are washed in a physiological buffer [e.g., phosphate-buffered saline (PBS) or Hank's buffer]. Single-cell suspensions are made by treating the tissue with a digestive enzyme (see below) or/and mincing and flushing the tissue parts through a nylon filter or by gentle pipetting (Falcon, Becton, Dickinson, San Jose, CA) with washing medium.

Isolated adherent cells from placenta tissue may be derived by treating the tissue with a digestive enzyme such as collagenase, trypsin and/or dispase; and/or effective concentrations of hyaluronidase or DNAse; and ethylenediaminetetra-acetic acid (EDTA); at temperatures between 25 - 50 °C, for periods of between 10 minutes to 3 hours. The cells may then be passed through a nylon or cheesecloth mesh filter of between 20 microns to 1 mm. The cells are then subjected to differential centrifugation directly in media or over a Ficoll or Percoll or other particulate gradient. Cells are centrifuged at speeds of between 100 to 3000 x g for periods of between 1 minutes to 1 hour at temperatures of between 4- 50 °C (see U.S. Pat. No. 7,078,230).

Cell retrieval is preferably effected under sterile conditions. Once isolated cells are obtained, they are allowed to adhere to an adherent material (e.g., configured as a surface) to thereby isolate adherent cells. Culturing may proceed under 2D conditions or may be further transferred to 3D conditions

As used herein "an adherent material" refers to a synthetic, naturally occurring or a combination of same of a non-cytotoxic (i.e., biologically compatible) material having a chemical structure (e.g., charged surface exposed groups) which may retain the cells on a surface.

Examples of adherent materials which may be used in accordance with this aspect of the invention include, but are not limited to, a polyester, a polypropylene, a polyalkylene, a polyfluorochloroethylene, a polyvinyl chloride, a polystyrene, a polysulfone, a cellulose acetate, a glass fiber, a ceramic particle, a matrigel, an extra cellular matrix component (e.g., fibronectin, vitronectin, chondronectin, laminin), a collagen, a poly L lactic acid, dextran and an inert metal fiber.

Further steps of purification or enrichment for a cell may be effected using methods which are well known in the art (such as by FACS using specific cell markers, as further described hereinabove).

Non-limiting examples of base media useful in culturing according to the invention include Minimum Essential Medium Eagle, ADC-1, LPM (Bovine Serum Albumin-free), F10(HAM), F12 (HAM), DCCM1, DCCM2, RPMI 1640, BGJ Medium (with and without Fitton-Jackson Modification), Basal Medium Eagle (BME-with the addition of Earle's salt base), Dulbecco's Modified Eagle Medium (DMEM-without serum), Yamane, IMEM-20, Glasgow Modification Eagle Medium (GMEM), Leibovitz L-15 Medium, McCoy's 5A Medium, Medium M199 (M199E-with Earle's sale base), Medium M199 (M199H-with Hank's salt base), Minimum Essential Medium Eagle (MEM-E-with Earle's salt base), Minimum Essential Medium Eagle (MEM-H-with Hank's salt base) and Minimum Essential Medium Eagle (MEM-NAA with non essential amino acids), among numerous others, including medium 199, CMRL 1415, CMRL 1969, CMRL 1066, NCTC 135, MB 75261, MAB 8713, DM 145, Williams' G, Neuman & Tytell, Higuchi, MCDB 301, MCDB 202, MCDB 501, MCDB 401, MCDB 411, MDBC 153. A preferred medium for use in the invention is DMEM. These and other useful media are available from GIBCO, Grand Island, N.Y., USA and Biological Industries, Bet HaEmek, Israel, among others. A number of these media are summarized in Methods in Enzymology, Volume LVIII, "Cell Culture", pp. 62 72, edited by William B. Jakoby and Ira H. Pastan, published by Academic Press, Inc.

The medium may be supplemented such as with serum such as fetal serum of human, bovine or other species, and optionally or alternatively, growth factors, vitamins (e.g. ascorbic acid), cytokines, salts (e.g. B-glycerophosphate), steroids (e.g. dexamethasone) and hormones e.g., growth hormone, erythropoeitin, thrombopoietin, interleukin 3, interleukin 6, interleukin 7, macrophage colony stimulating factor, c-kit ligand/stem cell factor, osteoprotegerin ligand, insulin, insulin like growth factors, epidermal growth factor, fibroblast growth factor, nerve growth factor, cilary neurotrophic factor, platelet derived growth factor, and bone morphogenetic protein at concentrations of between picogram/ml to milligram/ml levels.

It is further recognized that additional components may be added to the culture medium. Such components may be antibiotics, antimycotics, albumin, amino acids, and other components known to the art for the culture of cells. Additionally, components may be added to enhance the differentiation process when needed (see further below).

To maintain a xeno-free environment, the culture medium can be supplemented with a serum-replacement, human serum and/or synthetic or recombinantly produced factors.

As mentioned, once adherent cells are at hand they may be passaged to two dimensional or three dimensional settings. It will be appreciated though, that the cells may be transferred to a 3D-configured matrix immediately after isolation or alternatively, may be passaged to three dimensional settings following two dimensional conditions (as mentioned hereinabove).

Thus, the adherent material of this aspect of the invention is configured for 3D culturing thereby providing a growth matrix that substantially increases the available attachment surface for the adherence of the adherent cells so as to mimic the infrastructure of the tissue (e.g., placenta).

For high scale production, culturing can be effected in a 3D bioreactor.

Examples of such bioreactors include, but are not limited to, a plug flow bioreactor, a continuous stirred tank bioreactor, a stationary-bed bioreactor (packed bed bioreactor) and a fluidized bed bioreactor.

Furthermore, the cell cultures can be monitored for concentration levels of glucose, lactate, glutamine, glutamate and ammonium. The glucose consumption rate and the lactate formation rate of the adherent cells enable to measure cell growth rate and to determine the harvest time.

Other 3D bioreactors that can be used with the invention include, but are not limited to, a continuous stirred tank bioreactor, where a culture medium is continuously fed into the bioreactor and a product is continuously drawn out, to maintain a time-constant steady state within the bioreactor. A stirred tank bioreactor with a fibrous bed basket is available for example at New Brunswick Scientific Co., Edison, NJ), A stationary-bed bioreactor, an air-lift bioreactor, where air is typically fed into the bottom of a central draught tube flowing up while forming bubbles, and disengaging exhaust gas at the top of the column], a cell seeding perfusion bioreactor with Polyactive foams [as described in Wendt, D. et al., Biotechnol Bioeng 84: 205-214, (2003)] tubular poly-L-lactic acid (PLLA) porous scaffolds in a Radial-flow perfusion bioreactor [as described in Kitagawa et al., Biotechnology and Bioengineering 93(5): 947-954 (2006). Other bioreactors which can be used in accordance with the invention are described in U.S. Pat. Nos. 6,277,151, 6,197,575, 6,139,578, 6,132,463,5,902,741 and 5,629,186.

In an exemplary embodiment a total of 150 ± 50 x 10⁶ cells are seeded, 3-7 x 10⁶ cell / gr carrier are seeded, or 0.06-0.13 x 10⁶ cell / ml are seeded. According to an exemplary embodiment, cell seeding is effected at 1400-7000 cells/cm² FibraCel disks

Cells can be harvested when at least about 10 % of cells are proliferating while avoiding uncontrolled differentiation and senescence.

Culturing is effected for at least about 2 days, 3 days, 4 days, 5 days, 6 days, 7 days, 10 days, 14 days, 20 days, a month or even more. It will be appreciated that culturing in a bioreactor may prolong this period. Culturing of the adherent cells in the 3D culture can be effected under a continuous flow of a culture medium and/or under perfusion. Passaging may also be effected to increase cell number. It will be appreciated that culture medium may be changed in order to prolong and improve culturing conditions.

Adherent cells of placental tissue can be cryopreserved by suspending the cells in a cryopreservative such as DMSO, glycerol and propylene glycol. Cells can be cryopreserved after purification or culture. Typically, the cryopreservative is added in a stepwise fashion and the cells are slow cooled to -40 °C then stored at -196 °C. Cells may be rapidly thawed (e.g., in a 37 °C water bath) and assayed for the above criteria before use. Cryopreservation can allow for long-term storage of these cells for later transplantation or other purposes. Cryopreserving collections of purified populations of cells is particularly useful for producing a cell bank.

As mentioned, the population of cells of the present invention is selected by determining all of the aforementioned criteria.

According to a specific embodiment of the present invention selecting of the population is according to the following values:
(i) at least 70 % of viable cells in the candidate population; and
(ii) immune phenotype comprising a positive marker expression of CD73, CD29 and CD105 and a negative marker expression of CD45, CD14 and HLA-DR of cells in the candidate population, wherein cells comprising the positive marker expression make up at least 90 % of the candidate population and cells comprising the negative marker expression make up equally to- or less than 5 % of the candidate population.

Additionally, selection criteria of the population may further comprise
(iii) no xeno-contamination in the candidate population;
(iv) sterility of the candidate population; and
(v) immunosuppressive activity of cells in the candidate population;

Furthermore, the population is typically less committed to an osteogenic lineage as compared to adherent cells from bone marrow grown and allowed to differentiate under the same conditions.

Alternatively or in addition, the population is less committed to an adipogenic lineage as compared to adherent cells from bone marrow grown and allowed to differentiate under the same conditions.

Once qualified according to the above selection criteria, the population of adherent cells of a placenta tissue can be used for the treatment of ischemia.

The term "ischemia" as used herein refers to any pathology (disease, condition, syndrome or disorder) characterized by or associated with insufficient angiogenesis. Examples include, but are not limited to, a peripheral arterial disease (PAD) such as limb ischemia and critical limb ischemia (CLI), ischemic heart disease, ischemic brain disease (e.g. stroke), delayed wound-healing, delayed ulcer healing, reproduction associated disorders, arteriosclerosis, ischemic vascular disease, ischemic heart disease, myocardial ischemia, coronary artery disease (CAD), atherosclerotic cardiovascular disease, left main coronary artery disease, arterial occlusive disease, peripheral ischemia, peripheral vascular disease, vascular disease of the kidney, peripheral arterial disease, limb ischemia, lower extremity ischemia, cerebral ischemia, cerebro vascular disease, retinopathy, retinal repair, remodeling disorder, von Hippel-Lindau syndrome, hereditary hemorrhagic telengiectasiaischemic vascular disease, Buerger's disease, ischemic renal disease and ischemic placenta.

As used herein the term "treating" refers to inhibiting or arresting the development of a pathology (e.g., ischemia) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology. The term "treating" may also refer to alleviating or diminishing a symptom associated with the pathology.

As used herein the phrase "subject in need thereof" refers to any subject (e.g., mammal), such as a human subject who is diagnosed with or suffers from the pathology. Criteria for qualifying PAD patients for cell therapy in accordance with the present teachings are provided in Example 3 of the Examples section which follows.

Compositions of the present invention may, if desired, be presented in a pack or dispenser device, such as an FDA-approved kit, which may contain one or more unit dosage forms containing the active ingredient (e.g., cells). The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the invention formulated in a pharmaceutically acceptable carrier may also be prepared, placed in an appropriate container, and labeled for treatment of an indicated condition, as further detailed above.

The cells prepared according to the methods of the present invention can be administered to the subject per se, or in a pharmaceutical composition where it is mixed with suitable carriers or excipients.

As used herein, a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Hereinafter, the phrases "physiologically acceptable carrier" and "pharmaceutically acceptable carrier," which may be used interchangeably, refer to a carrier or a diluent that does not cause significant irritation to an organism and does not abrogate the biological activity and properties of the administered compound. An adjuvant is included under these phrases.

Herein, the term "excipient" refers to an inert substance added to a pharmaceutical composition to further facilitate administration of an active ingredient.

Techniques for formulation and administration of drugs may be found in the latest edition of "Remington's Pharmaceutical Sciences," Mack Publishing Co., Easton, PA.

Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer.

Pharmaceutical compositions suitable for use in the context of the present invention include compositions wherein the active ingredients are contained in an amount effective to achieve the intended purpose. More specifically, a "therapeutically effective amount" means an amount of active ingredients (e.g. cells) effective to prevent, alleviate, or ameliorate symptoms of a disorder or prolong the survival of the subject being treated.

Determination of a therapeutically effective amount is well within the capability of those skilled in the art, especially in light of the detailed disclosure provided herein.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration, and dosage can be chosen by the individual physician in view of the patient's condition. (See, e.g., Fingl, E. et al. (1975), "The Pharmacological Basis of Therapeutics," Ch. 1, p.1.)

Depending on the severity and responsiveness of the condition to be treated, dosing can be of a single or a plurality of administrations. Commonly, however, subjects receive a single transplant, with multiple transplants being extremely rare. However, the amount of a composition to be administered will, of course, be dependent on the subject being treated, the severity of the affliction, the manner of administration, the judgment of the prescribing physician, etc.

It is expected that during the life of a patent maturing from this application many relevant three dimensional cultures will be developed and the scope of the term three dimensional cultures is intended to include all such new technologies *a priori.*

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### 3D ADHERENT CELLS MANUFACTURED BY PLURIX COMPARED TO 3D ADHERENT CELLS MANUFACTURED BY CELLIGEN

In order to provide large scale 3D adherent cells, two manufacturing systems were utilized referred to herein as Plurix (teachings of WO/2007/108003) and Celligen (teachings of the present invention).

### Materials and Experimental Methods

### Production of 3D adherent cells (PLX) by PluriX™ Plug Flow bioreactor

As described in WO/2007/108003. In short, the process starts by collection of a placenta from a planned cesarean delivery at term. Inner parts of a full-term delivery placenta (Bnei Zion medical center, Haifa, Israel) were cut under aseptic conditions, washed 3 times with Hank's Buffer and incubated for 3 hours at 37 °C with 0.1 % Collagenase (1mg / ml tissue; Sigma- Aldrich, St. Lewis, MO). Using gentle pipetting, suspended cells were then washed with DMEM supplemented with 10 % FCS, Pen-Strep-Nystatin mixture (100 U/ml:100 µg/m1:1.25 un/ml) and 2 mM L-glutamine, seeded in 75 cm² flasks and incubated at 37 °C in a tissue culture incubator under humidified condition with 5 % CO₂.

### Two dimensional (2D) cell growth

Cells were allowed to adhere to a plastic surface for 72 hours after which the media was changed every 3-4 days. After 2-3 passages, the cells were cryopreserved, thawed and seeded for a secondary growth in flasks. When reaching 60-80 % confluence (usually 10-12 days), cells were detached from the growth flask using 0.25 % trypsin-EDTA and seeded into new flasks. Cultured cells were thereafter collected for analysis or for culturing in bioreactors.

### PluriX™ Plug Flow bioreactor

The PluriX™ Plug Flow bioreactor (Pluristem, Haifa, Israel; as illustrated in U.S. Pat. No. 6,911,201 and WO/2007/108003), was loaded with 1-100 ml packed 3D porrosive carriers (4 mm in diameter) made of a non woven fabric matrix of polyester. These carriers enable the propagation of large cell numbers in a relatively small volume. Glassware was designed and manufactured by Pluristem (Pluristem, Haifa, Israel). The bioreactor was maintained in an incubator of 37 °C, with flow rate regulated and monitored by a valve and peristaltic pump. The bioreactor contains a sampling and injection point, allowing the sequential seeding of cells. Culture medium was supplied at pH 6.7-7.4 from a reservoir. The reservoir was supplied by a filtered gas mixture, containing air/CO₂/O₂ at differing proportions, depending on cell density in the bioreactor. The O₂ proportion was suited to the level of dissolved O₂ at the bioreactor exit, determined by a monitor. The gas mixture was supplied to the reservoir via silicone tubes or diffuser (Degania Bet, Emek Hayarden, Israel). The culture medium was passed through a separating container which enables collection of circulating, non-adherent cells. Circulation of the medium was obtained by a peristaltic pump. The bioreactor was further equipped with an additional sampling point and containers for continuous medium exchange.

### Production of 3D-adherent cells (PLX)

Non-confluent primary human adherent 2D cell cultures, grown as described above, were trypsinized, washed, resuspended in DMEM supplemented with 10 % FBS, Pen-Strep-Nystatin mixture (100 U/ml:100 ug/ml:1.25 un/ml) and 2 mM L-glutamine, and seeded (10³-10⁵ cells/ml) via an injection point onto the 3D carriers in a sterile Plug Flow bioreactor. Prior to inoculation, bioreactor was filled with PBS-Ca-Mg (Biological Industries, Beit Ha'emek, Israel), autoclaved (120 °C, 30 min) and washed with Dulbecco's growth medium containing 10 % heat-inactivated fetal calf serum and a Pen-Strep-Nystatin mixture (100 U/ml:100 ug/ml:1.25 un/ml). Flow was kept at a rate of 0.1-5 ml/min. Seeding process involved cease of circulation for 2- 48 hrs, thereby allowing the cells to settle on the carriers. Bioreactor was kept under controlled temperature (37 °C) and pH conditions (pH = 6.7-7.4); using an incubator supplied with sterile air and CO₂ as needed. Growth medium was replaced 2-3 times a week. Circulation medium was replaced with fresh DMEM media, every 4 hr to 7 days. At a density of 1 x 10⁶-1 x 10⁷ cells/ml (following 12-40 days of growth), total medium volume was removed from the bioreactor and bioreactor and carriers were washed 3-5 times with PBS. 3D-adherent cells were then detached from the carriers with Trypsin-EDTA; (Biological Industries, Beit Ha'emek, Israel; 3-15 minutes with gentle agitation, 1-5 times), and were thereafter resuspended in DMEM and cryopreserved.

***Production of 3D adherent cells by Celligen*™ *Plug Flow bioreactor (PLX-C)-***The production of adherent cells by the present teachings utilizes Celligen^{™} (PLX-C cells). The process starts by collection of a placenta from a planned caesarean section at term.

Adherent cells are then isolated from whole placenta tissue, grown in tissue culture flasks (2D cultures), harvested and stored in liquid nitrogen as 2D-Cell Stock (2DCS), the appropriate amount of 2DCS are thawed, washed and seeded onto carriers in bioreactors for further expansion as 3D-culture. After 4-12 days of growth in the bioreactors, cells are harvested and cryopreserved in gas phase of liquid nitrogen as PLX-C.

### Receipt of Human Tissue

All placentas obtained were received from the maternity ward under approval of the Helsinki Committee of the medical facility. Accordingly, all placenta donors signed an informed consent and Donor Screening and Donor Testing was performed (IPC1). Immediately after taking the placenta from the donor (during the caesarean procedure), it was placed in a sterile plastic bag and then in a temperature-preserving box with ice packs.

### Recovery and Processing of adherent cells

To initiate the process, the placenta was cut into pieces under aseptic conditions under laminar flow hood, washed with Hank's buffer solution and incubated for 3 hours at 37 °C with 0.1 % Collagenase (1 mg Collagenase/ml tissue). 2D cell medium (2D-Medium comprising DMEM supplemented with 10 % FBS, fungizone 0.25 µg/ml and Gentamycine 50 µg/ml) was added and the digested tissue was roughly filtered through a sterile metal strainer, collected in a sterile beaker and centrifuged (10 minutes, 1200 RPM, 4 °C). Using gentle pipeting, suspended cells were then diluted with 2D-Medium supplemented with antibiotics, seeded in 175 cm² flasks and incubated at 37 °C in a tissue culture incubator under humidified condition supplemented with 5 % CO₂. Following 2-3 days, in which the cells were allowed to adhere to the flask surface, they were washed with PBS and 2D-Medium was added.

### Two Dimensional (2D) Cell Growth

Prior to the first passage, growth medium samples of 10 % of the total flask number in quarantine was pooled and taken for mycoplasma testing (IPC2). If cells were found to be negative for Mycoplasma (EZ-PCR Mycoplasma kit, Biological Industries, Israel), cells were released from quarantine. After 1-2 additional passages, cells were transferred to the 2D production clean room (2DP). Once in Room 2DP, culture was continued for another 3-6 passages. Throughout the process, cultures were grown in 2D-Medium without antibiotics in a tissue culture incubator under humidified conditions with 5 % CO₂ at 37 °C. After a total of 6-9 passages (9-17 cell doublings), cells were collected and cryopreserved as the 2D-Cell Stock (2DCS).

The first passage was usually carried out after 7-15 days. Beginning at passage 2 and continuing until passage 6-8, cells were passaged when the culture reached 70-90 % confluence, usually after 4-5 days (1.5-2 doublings). The cells were detached from the flasks using 0.25 % trypsin-EDTA (4 minutes at 37 °C) and seeded in a culture density of 4 ± 0.5 x 10³ cells/cm². The size of the tissue culture flasks raised as the passages proceed. The culturing process started in 175 cm² tissue culture flask, continued in 500 cm² (Triple flask) and finally the cells were seeded into Cell Factory 10 tray (6320 cm²).

Prior to cryopreservation, at the end of 2DCS growth period, the growth medium was collected and the sample was prepared to be sent to an approved GLP laboratory for Mycoplasma test (IPC 4).

### Cryopreservation Procedure for 2D-Cell-Stock Product

For 2DCS cryopreservation, 2D-cultured cells were collected under aseptic conditions using 0.25 % trypsin-EDTA. The cells were centrifuged (1200 RPM, 10', 4 °C), counted and re-suspended in 2D-Medium.

For freezing, cell suspensions were diluted 1:1 with 2D-Freezing Mixture (final concentrations was 10 % DMSO, 40 % FBS and 50 % 2D-Medium). Approximately 1.5 - 2.5 x 10⁹ cells were manufactured from one placenta. 4 ml of the cells were stored at a final concentration of 10 x 10⁶/ ml in 5ml cryopreservation polypropylene vials. The vials were labeled and transferred to a controlled rate freezer for a graduated temperature reducing process (1 °C/min), after which they were transferred to storage in gas-phase of a liquid nitrogen freezer. This material was referred to as the 2D-Cell Stock (2DCS) batch.

### Initiation of the Three Dimensional (3D) Culture Procedures

To begin 3D culture, an appropriate amount (150 ± 50 x 10⁶) of cells from 2DCS were thawed in the 2DP room and washed with 3D-Medium (DMEM with 10 % FBS and 20 Mm Hepes) to remove DMSO prior to seeding in the prepared-in-advanced bioreactor systems. The content of each 2DCS vial was pipetted and diluted 1:9 with pre-warmed (37 °C) 3D-Medium. The cells were centrifuged (1200 RPM, 10', 4 °C) and re-suspended again in 50-100 ml pre-warmed (37 °C) 3D-Medium in a 250 ml sterile bottle. A sample was taken and cells were counted using a Trypan Blue stain in order to determine cell number and viability. The cell suspension was transferred under a laminar flow hood into a 0.5 L seeding bottle. From the seeding bottle the cell suspension was transferred via sterile tubing to the bioreactor by gravitation.

### Production of 3D-adherent cells (PLX-C) in Celligen Bioreactor

### Bioreactor Description

3D growth phase was performed using an automatic CelliGen Plus@ or BIOFLO 310 bioreactor system [(New Brunswick Scientific (NBS)]. The bioreactor system was used for cultivation of cell culture, in which conditions were suitable for high cell concentrations. The cultivation process was carried out using a bioreactor in a perfusion mode. The lab scale bioreactor was constructed of two main systems - the control system and the bioreactor itself (vessel and accessories). The parameters of the process were monitored and controlled by a control console which included connectors for probes, motor and pumps, control loops for Dissolved Oxygen (DO), pH, perfusion and agitation (with a motor), a gases control system, water circulation and heating system for temperature control and an operator interface. The controlled process parameters (such as temperature, pH, DO etc.) could be displayed on the operator interface and monitored by a designated controller.

### Cell culture growth procedure in the bioreactors

As noted in the section hereinabove, 150 ± 50 x 10⁶ cells from the cryopreserved 2DCS were thawed, washed and seeded in a sterile bioreactor. The bioreactor contained 30-50 gr carriers (FibraCel® disks, NBS), made of Polyester and Polypropylene and 1.5 ± 0.1 L 3D-Medium. The growth medium in the bioreactor was kept at the following conditions: 37 °C, 70 % Dissolved Oxygen (DO) and pH 7.3. Filtered gases (Air, CO₂, N₂ and O₂) were supplied as determined by the control system in order to keep the DO value at 70 % and the pH value at 7.3. For the first 24 hours, the medium was agitated at 50 Rounds Per Minutes (RPM) and increased up to 200 RPM by day 2. For the first 2-3 days, the cells were grown in a batch mode. Perfusion was initiated when the medium glucose concentration decreased below 550 mg/liter. The medium was pumped from the feeding container to the bioreactor using sterile silicone tubing. All tubing connections were performed under laminar flow using sterile connectors. The perfusion was adjusted on a daily basis in order to keep the glucose concentration constant at approximately 550±50 mg\liter. A sample of the growth medium was taken every 1-2 days for glucose, lactate, glutamine, glutamate and ammonium concentration determination (BioProfile 400 analyzer, Nova Biomedical). The glucose consumption rate and the lactate formation rate of the cell culture enabled to measure cell growth rate. These parameters were used to determine the harvest time based on accumulated experimental data.

### Harvest of the 3D Grown Cells from the Bioreactor

The cell harvest process started at the end of the growth phase (4-12 days). Two samples of the growth medium were collected. One sample was prepared to be sent to an approved GLP laboratory for Mycoplasma testing according to USP and Eu standards. This medium samples was considered as part of the Mycoplasma testing of the final product and the results were considered as part of the criteria for product release.

The 3D-grown culture was harvested in the Class-100 laminar area in room 3DP as follows:
The bioreactor vessel was emptied using gravitation via tubing to a waste container. The bioreactor vessel was then refilled with 1.5 L pre-warmed PBS (37°C). The agitation speed was increased to 150 RPM for 2 minutes. The PBS was drained via tubing by pressure or gravity to the waste bottle. The washing procedure was repeated twice.

In order to release the cells from the carriers, 1.5 L pre-warmed to 37°C Trypsin-EDTA (Trypsin 0.25 %, EDTA 1 mM) was added to the bioreactor vessel and carriers were agitated for 1-4 minutes in 150 RPM, 37 °C. 250 ml FBS (Fetal Bovine Serum) was added to the bioreactor vessel and the cell suspension was collected to a 5 L sterile container. Cell suspension was divided to 4 500 ml sterile centrifuge tubes, which were centrifuged (1200 RPM, 10 min, 4 °C) and resuspended in a cryopreservation solution at a concentration of 5-30x10⁶ cells/ml. Cells were aseptically filled and cryopreserved as PLX-C.

### Cell Cycle analysis

PLX-C cells obtained by Celligen and PLX cells obtained by Plurix were fixed with 70 % EtOH O.N, centrifuged and re-suspended in a Propidium Iodide (PI) solution containing 2 µg/ml PI (Sigma), 0.2 mg/ml Rnase A (Sigma) and 0.1 % (v/v) Triton (Sigma) for 30 minutes.. Cell cycle was analyzed by FACS.

### Gene expression array (Microarray)

Adherent cells were obtained from human full term placentas and were expanded Plurix or by Celligen. Three different batches of cells were obtained from each of the expansion methods for further examination.

RNA was extracted from the cells (Qiagen- Rneasy micro kit) and applied to an Affymetrix whole genome expression array GeneChip® Human Exon 1.0 ST Array (Affymetrix, Santa Clara, California, USA).

***FACS analysis of membrane markers -*** cells were stained with monoclonal antibodies as previously described. In short, 400,000-600,000 cells were suspended in 0.1 ml flow cytometer buffer in a 5 ml test tube and incubated for 15minutes at room temperature (RT), in the dark, with each of the following monoclonal antibodies (MAbs): FITC-conjugated anti-human CD29 MAb (eBioscience), PE conjugated anti human CD73 MAb (Becton Dickinson) ,PE conjugated anti human CD105 MAb (eBioscience), PE conjugated anti human CD90 MAb (Becton Dickinson), FITC-conjugated anti-human CD45 MAb (IQProducts), PE-conjugated anti-human CD19 MAb (IQProducts), PE conjugated anti human CD14 MAb (IQProducts), FITC conjugated anti human HLA-DR MAb (IQProduct), PE conjugated anti human CD34 MAb (IQProducts), FITC conjugated anti human CD31 MAb (eBioscience), FITC conjugated anti human KDR MAb (R&D systems), anti human fibroblasts marker (D7-FIB) MAb(ACRIS, FITC-conjugated anti-human CD80 MAb (BD), FITC-conjugated anti-human CD86 MAb (BD), FITC-conjugated anti-human CD40 MAb (BD), FITC-conjugated anti-human HLA-ABC MAb (BD), Isotype IgG1 FITC conjugated (IQ Products), Isotype IgG1 PE conjugated (IQ Products).

Cells were washed twice with flow cytometer buffer, resuspended in 500 µl flow cytometer buffer and analyzed by flow cytometry using FC-500 Flow Cytometer (Beckman Coulter). Negative controls were prepared with relevant isotype fluorescence molecules.

### Mixed Lymphocyte Reaction (MLR)

2 x 10⁵ peripheral blood (PB) derived MNC (from donor A) were stimulated with equal amount of irradiated (3000 Rad) PB derived MNCs (from donor B). Increasing amounts of PLX-Cs were added to the cultures. Three replicates of each group were seeded in 96-well plates. Cells were cultured in RPMI 1640 medium containing 20 % FBS. Plates were pulsed with 1 µC ³H-thymidine during the last 18 hr of the 5-day culturing. Cells were harvested over a fiberglass filter and thymidine uptake was quantified with scintillation counter.

For CFSE staining, PB-MNC cells were stained for CFSE (Molecular Probes) for proliferation measurement before culturing. Cells were collected after 5 days and the intensity of CFSE staining was detected by Flow Cytometry.

### ELISA

ELISA was carried out as was previously described. In short, MNCs (isolated from peripheral blood) were stimulated with 5 µg/ml ConA (Sigma), 0.5 µg/ml LPS (SIGMA) or 10 µg/ml PHA (SIGMA) in the presence of PLX-C under humidified 5 % CO₂ atmosphere at 37 °C. Supernatants were collected and subjected to cytokine analysis using ELISA kits for IFNγ (DIACLONE), TNFα (DIACLONE) and IL-10 (DIACLONE).

### Experimental results

The changes in manufacturing with Celligen as compared to Plurix resulted in several major differences (summarized in Table 1, below).

**Table 1: Comparison between Plurix system (WO/2007/108003) and Celligen system (teachings of the present invention)**

| **Parameter** | WO/2007/1 08003 | **Teachings of the present invention** | **Improvement** |
|---|---|---|---|
| Working volume (ml) | 280 | 1500 | Scale up of the process. Higher production level in the present teachings (2-8 population doubling) |
| Weight of carrier (gr) | 1.4 | 30 | Scale up of the process in the present teachings. |
| Bed configuration | Conic, 50 ml column | Cylinder Packed bed | The present teachingsBetter flow of medium and nutrients. WO/2007/1080 03 - Inefficient flow due to narrow outlet form the conic structure Better homogeneity of medium flow. Channeling in the plurix |
| Cell concentration at seeding (cell / gr carrier) | 3 x 10⁶ cell / gr carrier | 5 x 10⁶ cell / gr carrier | Better cell to cell interaction in the present teachings |
| Cell concentration at seeding (cell / ml) | 0.015 x 10⁶ cell / ml | 0.1 x 10⁶ cell / ml | Better cell to cell interaction in the present teachings |
| Seeding procedure | Seeding at low medium volume for 24h followed by addition of medium to final working volume | Seeding at the final working volume while agitating | WO/2007/1080 03 - Heterogenic distribution of the cell culture inside the carrier bed Insufficient medium volume in the first 24 h of the run. Leading to unsuitable working conditions (acidic environment) |
| Production phase duration | 14-21 days | 4-12 days | Better product quality. Efficient harvest process. Better yield. Lower cost process in the present teachings |
| Mode of operation | Repeated batch - medium change twice a week | Perfusion mode - rate was adjusted according to the glucose concentration (the medium was changed at glucose concentration of 550 ± 50 mg/L) | Present teachings - Moderate changes of the conditions regarding medium composition throughout the run Continuous removal of toxic agents produced by the cells. In batch mode - lower concentration of essential nutrients (limiting factors) Less cell debris |
| Harvest procedure | Harvesting in 50 ml tubes Trypsinizati on 3 cycles | Harvesting inside the bioreactor Trypsinization 1 cycle | Present teachings - More efficient process Harvest is carried out in a close system. 1 trypsinization cycle - better quality of the cells. |
| Agitation | medium Circulation between reservoir container to the column using peristaltic pump | Cell lift impeller | Present teachings - Medium is flowing through the packed bed -Better supply of nutrients and oxygen to the culture. Homogeneity of the medium Improves other control loops (temp., DO, pH) |
| Temperature control | The production was carried out inside an incubator. Indirect temperature control (of the incubator chamber). Heat transfer via air interface | On-line direct control. Heat transfer via water jacket. | Present teachings - more accurate measurement of the culture temperature. Quick response. Short time to reach set point. |
| Temperature monitoring | Manually. Indirect water temperature monitoring. | On-line direct monitoring. | Present teachings - Better monitoring and control of the process. Quick response to malfunctions. |
| DO monitoring | None | On-line monitoring | Present teachings - Better monitoring and control of the process. Quick response to malfunctions |
| DO control | None. Introduction of air only | On-line direct control of a specific set point using Air, O₂ and N₂. | Present teachings Better control of DO level. Better maintenance of a specified working conditions |
| pH monitoring and control | Only visual monitoring (Phenol red as part of the medium) | On-line Control and monitoring | Present teachings - Better control of pH level. Better maintenance of a specified working conditions |
| Aeration | Sparge only | Overlay (sparge as an option) | WO/2007/1080 03 - Aeration by sparge creates foam that might damage the cells. |

The changes in the manufacturing process resulted in changes in characteristics of the obtained 3D adherent cells. These differences are summarized below.

***Cell cycle analysis of PLX manufactured by Plurix compared to PLX***-***C manufactured by Celligen** -* PLX-C cells obtained by the present teachings (by the Celligen system) were compared to PLX cells obtained by Plurix (WO/2007/108003) in order to examine the distribution of the cells between the different phases of the cell cycle. As is clear from Figures 1A-B, PLX-C cells expanded by Celligen exhibited typical proliferating profile (distribution of cells between the different phases of cell cycle). Specifically, 28 % of cells were in S and G2/M phases (Figure 1A). These results indicated that cells were harvested during proliferation and that the Celligen bioreactor conditions supported cell growth.

***Microarray comparison between Plurix and Celligen obtained cells** -* gene expression arrays enabled to simultaneously monitor genome-wide expression profiles of adherent cells derived from human full term placentas expanded by Plurix (PLX) or by Celligen (PLX-C). These results enabled to asses the molecular mechanism underlying phenotypic variation between cells obtained by these different growth methods (see Table 2, below).

**Table 2: Gene expression in Plurix cells (WO/2007/108003) compared to Celligen cells (teachings of the present invention)**

| **Gene** | **Celligen vs Plurix (fold change)** | **p-value(treat)** |
|---|---|---|
| interferon-induced protein with tetratricopeptide repeats | **17.52** | 0.0401812 |
| aldehyde dehydrogenase 1 family, member A1 | **16.76** | 0.00145807 |
| leukocyte-derived arginine aminopeptidase | **13.99** | 3.88E-06 |
| keratin 27 pseudogene 27 | **12.25** | 0.000224998 |
| similar to Keratin, type I cytoskeletal 18 (Cytokerati | **11.83** | 0.000304949 |
| G protein-coupled receptor, family C, group 5, member A | **10.35** | 3.39E-05 |
| integrin, alpha 6 | **9.84** | 0.0411667 |
| G protein-coupled receptor 126 | **8.73** | 0.00197635 |
| coagulation factor III (thromboplastin, tissue factor) | **7.36** | 0.012192 |
| Rho GDP dissociation inhibitor (GDI) beta | **7.36** | 0.00200066 |
| signal peptide, CUB domain, EGF-like 3 | **7.20** | 0.0255115 |
| interferon-induced protein with tetratricopeptide repeats | **7.09** | 0.0139777 |
| dickkopf homolog 1 (Xenopus laevis) | **7.06** | 3.06E-07 |
| NAD(P)H dehydrogenase, quinone 1 | **6.63** | 0.000282423 |
| keratin 18 | **6.46** | 0.000514523 |
| opioid growth factor receptor-like 1 | **5.96** | 0.00114551 |
| mal, T-cell differentiation protein-like | **5.95** | 0.00664216 |
| neurofilament, medium polypeptide 150kDa | **5.86** | 0.0190611 |
| DEP domain containing 1 | **5.82** | 0.000370513 |
| cathepsin C | **5.72** | 0.00532262 |
| WAS | **5.47** | 0.00178153 |
| serpin peptidase inhibitor, clade B (ovalbumin), member | **5.44** | 0.0190218 |
| solute carrier family 7, (cationic amino acid transporte | **5.33** | 0.00688017 |
| interferon-induced protein with tetratricopeptide repea | **5.18** | 0.00357376 |
| NUF2, NDC80 kinetochore complex component, homolog (S. cere | **5.05** | 0.00276524 |
| SHC SH2-domain binding protein 1 | **4.95** | 0.00430878 |
| thioredoxin reductase 1 | **4.86** | 0.000197486 |
| lung cancer metastasis-associated protein | **4.85** | 0.00148024 |
| Rho GTPase activating protein 29 | **4.85** | 0.0466211 |
| cell division cycle 20 homolog (S. cerevisiae) | **4.80** | 0.00514206 |
| family with sequence similarity 111, member B | **4.63** | 0.000125819 |
| PDZ binding kinase | **4.54** | 0.00784983 |
| establishment of cohesion 1 homolog 2 (S. cerevisiae) | **4.53** | 0.000773033 |
| guanylate binding protein 4 | **4.47** | 0.000215944 |
| lipase A, lysosomal acid, cholesterol esterase (Wolman dise | **4.42** | 0.0167385 |
| kinesin family member 20A | **4.39** | 0.00582352 |
| KIAA0101 | **4.28** | 0.0105909 |
| cyclin-dependent kinase inhibitor 3 (CDK2-associated dual | **4.25** | 0.000732492 |
| thymidylate synthetase | **4.23** | 0.00685584 |
| chromosome 13 open reading frame 3 | **4.18** | 0.000548296 |
| aurora kinase A | **4.16** | 0.00632571 |
| nei endonuclease VIII-like 3 (E. coli) | **4.14** | 0.00115606 |
| centrosomal protein 55kDa | **4.13** | 0.0021952 |
| oxidized low density lipoprotein (lectin-like) receptor 1 | **4.11** | 0.0205198 |
| denticleless homolog (Drosophila) | **4.05** | 0.00141153 |
| anillin, actin binding protein | **4.01** | 0.010923 |
| ribonucleotide reductase M2 polypeptide | **3.98** | 0.00834059 |
| ankyrin repeat domain 1 (cardiac muscle) | **3.93** | 0.00911953 |
| transcription factor 19 (SC1) | **3.89** | 0.00109627 |
| keratin 18 | **3.89** | 0.000112551 |
| non-SMC condensin I complex, subunit G | **3.88** | 0.00537097 |
| cyclin E2 | **3.87** | 0.000203389 |
| trypsinogen C | **3.86** | 0.00416276 |
| small nucleolar RNA, C | **3.81** | 0.0334484 |
| tight junction protein 2 (zona occludens 2) | **3.81** | 0.00012562 |
| kinesin family member 18A | **3.78** | 0.00134108 |
| kinesin family member 2C | **3.77** | 0.0059888 |
| shugoshin-like 1 (S. pombe) | **3.76** | 0.00101318 |
| polo-like kinase 1 (Drosophila) | **3.75** | 0.0140309 |
| thymidine kinase 1, soluble | **3.73** | 0.00124134 |
| transcription factor 19 (SC1) | **3.73** | 0.00124327 |
| transcription factor 19 (SC1) | **3.73** | 0.00124327 |
| claspin homolog (Xenopus laevis) | **3.71** | 0.00683624 |
| GINS complex subunit 1 (Psf1 homolog) | **3.69** | 0.00104515 |
| microsomal glutathione S-transferase 1 | **3.67** | 0.041701 |
| arylacetamide deacetvlase-like 1 | **3.67** | 0.000902645 |
| SPC25, NDC80 kinetochore complex component, homolog (S. ce | **3.65** | 0.00568662 |
| integrin, alpha 4 (antigen CD49D, alpha 4 subunit of VLA-4 | **3.62** | 0.0158411 |
| catenin (cadherin-associated protein), alpha-like 1 | **3.57** | 7.46E-05 |
| discs, large homolog 7 (Drosophila) | **3.56** | 0.0317074 |
| v-myb myeloblastosis viral oncogene homolog (avian)-lik | **3.55** | 0.0043878 |
| serglycin | **3.54** | 0.0443487 |
| centromere protein N | **3.53** | 0.000540143 |
| cyclin A2 | **3.53** | 0.00965934 |
| heat shock 22kDa protein 8 | **3.52** | 0.0219583 |
| sema domain, immunoglobulin domain (Ig), short basic doma | **3.49** | 0.008548 |
| Rho GTPase activating protein 11A | **3.49** | 0.00834174 |
| Fanconi anemia, complementation group I | **3.43** | 0.00464532 |
| BUB1 budding uninhibited by benzimidazoles 1 homolog (yeast | **3.42** | 0.0108258 |
| ovary-specific acidic protein | **3.42** | 0.00334641 |
| cholinergic receptor, muscarinic 2 | **3.41** | 0.0320078 |
| cell division cycle 2, G1 to S and G2 to M | **3.41** | 0.0017111 |
| protein regulator of cytokinesis 1 | **3.39** | 0.0325664 |
| minichromosome maintenance complex component 5 | **3.38** | 0.00475504 |
| sperm associated antigen 5 | **3.37** | 0.00906321 |
| maternal embryonic leucine zipper kinase | **3.34** | 0.00908391 |
| small nucleolar RNA, C | **3.33** | 0.0298703 |
| carnitine palmitoyltransferase 1A (liver) | **3.33** | 0.00170894 |
| similar to Ubiquitin-conjugating enzyme E2S (Ubiqui | **3.33** | 0.000415822 |
| kinesin family member 11 | **3.33** | 0.00915145 |
| NIMA (never in mitosis gene a)-related kinase 7 | **3.33** | 0.00159114 |
| ADAM metallopeptidase with thrombospondin type 1 motif, | **3.32** | 0.0102751 |
| transforming, acidic coiled-coil containing protein 3 | **3.31** | 0.0014577 |
| cyclin B1 | **3.29** | 0.0103092 |
| MAD2 mitotic arrest deficient-like 1 (yeast) | **3.28** | 0.00488102 |
| dihydrofolate reductase | **3.28** | 0.00178879 |
| NIPA-like domain containing 3 | **3.27** | 0.00164708 |
| cell division cycle associated 2 | **3.26** | 0.0122226 |
| apolipoprotein B mRNA editing enzyme, catalytic polypep | **3.26** | 0.00308692 |
| cyclin B2 | **3.25** | 0.016544 |
| endonuclease domain containing 1 | **3.24** | 0.000429245 |
| dihydrofolate reductase pseudogene ATPase, Na+ | **3.23** | 0.00141306 |
| | **3.23** | 0.000381464 |
| replication factor C (activator 1) 3, 38kDa | **3.23** | 0.00109668 |
| WD repeat domain 76 | **3.22** | 0.0023531 |
| pleckstrin 2 | **3.17** | 0.0304429 |
| Rac GTPase activating protein 1 | **3.17** | 0.00381613 |
| PHD finger protein 19 | **3.17** | 0.000177604 |
| deleted in lymphocytic leukemia, 2 | **3.15** | 0.0109528 |
| centromere protein I | **3.15** | 0.0106816 |
| BRCA1 associated RING domain 1 | **3.14** | 0.000540414 |
| regulator of G-protein signalling 4 | **3.13** | 0.00781061 |
| STAM binding protein-like 1 | **3.11** | 0.0181743 |
| sulfiredoxin 1 homolog (S. cerevisiae) | **3.10** | 5.14E-05 |
| chromosome 15 open reading frame 23 | **3.08** | 0.000147331 |
| TTK protein kinase | **3.08** | 0.0112171 |
| non-SMC condensin II complex, subunit G2 | **3.08** | 0.0130322 |
| villin 2 (ezrin) | **3.07** | 0.0131934 |
| stomatin | **3.06** | 0.00387095 |
| protein tvrosine phosphatase-like A domain containing | **3.06** | 0.0419644 |
| serpin peptidase inhibitor, clade B (ovalbumin), member | **3.05** | 0.0030439 |
| kinesin family member 4A | **3.05** | 0.0114203 |
| hypothetical protein DKFZp762E1312 | **3.05** | 0.00726778 |
| ubiquitin-conjugating enzyme E2S | **3.04** | 0.00118205 |
| hydroxysteroid dehydrogenase like 2 | **3.03** | 3.71E-05 |
| ATPase family, AAA domain containing 2 | **3.01** | 0.00415258 |
| TPX2, microtubule-associated, homolog (Xenopus laevis) | **3.00** | 0.0253137 |
| histone cluster 1, H4d | **3.00** | 0.030183 |
| kinesin family member 23 | **2.99** | 0.00790585 |
| heat shock 70kDa protein 2 | **2.99** | 0.0215102 |
| origin recognition complex, subunit 1-like (yeast) | **2.99** | 0.00207753 |
| dihydrofolate reductase | **2.98** | 0.00307793 |
| hvaluronan-mediated motility receptor (RHAMM) | **2.97** | 0.00467816 |
| 3'-phosphoadenosine 5'-phosphosulfate synthase 2 | **2.97** | 1.43E-05 |
| glycerol-3-phosphate dehydrogenase 2 (mitochondrial) | **2.95** | 0.00211969 |
| nucleolar and spindle associated protein 1 | **2.95** | 0.00520875 |
| diaphanous homolog 3 (Drosophila) | **2**.**95** | 0.00107709 |
| kinesin family member 14 | **2.94** | 0.00947901 |
| histone cluster 1, H1b | **2.93** | 0.0470898 |
| guanine nucleotide binding protein (G protein), alpha inhi | **2.92** | 0.00184597 |
| minichromosome maintenance complex component 8 | **2.92** | 0.000841489 |
| cancer susceptibility candidate 5 | **2.92** | 0.0330594 |
| leukotriene B4 12-hydroxydehydrogenase | **2.92** | 0.000685452 |
| glutamate-cysteine ligase, modifier subunit | **2.91** | 0.00378868 |
| forkhead box M1 | **2.91** | 0.0203154 |
| adipose differentiation-related protein | **2.90** | 0.000331751 |
| membrane bound O-acyltransferase domain containing 1 | **2.90** | 0.01185 |
| ubiquitin-conjugating enzyme E2T (putative) | **2.90** | 0.00741886 |
| cell division cycle associated 3 | **2.89** | 0.006289 |
| integrin, alpha 3 (antigen CD49C, alpha 3 subunit of VLA-3 | **2.88** | 0.00574148 |
| coagulation factor XIII, B polypeptide | **2.88** | 0.0294465 |
| RAD51 homolog (RecA homolog, E. coli) (S. cerevisiae) | **2.87** | 0.000854739 |
| ATP-binding cassette, sub-family C (CFTR | **2.87** | 0.00382491 |
| family with sequence similarity 29, member A | **2.85** | 0.00111165 |
| SH2 domain containing 4A | **2.84** | 0.0323646 |
| membrane protein, palmitoylated 1, 5kDa | **2.84** | 0.000396285 |
| CDC28 protein kinase regulatory subunit 1B | **2.84** | 0.0107391 |
| PSMC3 interacting protein | **2.84** | 0.00766442 |
| elastin microfibril interfacer 2 | **2.84** | 0.0192072 |
| topoisomerase (DNA) II alpha 170kDa | **2.83** | 0.0321109 |
| transmembrane protein 106C | **2.82** | 0.000214223 |
| histone cluster 1, H3b | **2.80** | 0.0304598 |
| chromosome 18 open reading frame 24 | **2.80** | 0.00347442 |
| epidermal growth factor receptor pathway substrate 8 | **2.79** | 0.0194949 |
| high-mobility group nucleosomal binding domain 2 | **2.78** | 0.0030536 |
| SCL | **2.78** | 0.00390288 |
| hect domain and RLD 4 | **2.78** | 0.00679184 |
| ASF1 anti-silencing function 1 homolog B (S. cerevisiae) | **2.77** | 0.00543408 |
| thyroid hormone receptor interactor 13 | **2.76** | 0.0118319 |
| cell division cycle associated 8 | **2.75** | 0.00619878 |
| kinesin family member C1 | **2.74** | 0.00821937 |
| high-mobility group nucleosomal binding domain 2 | **2.73** | 0.00384071 |
| ornithine decarboxylase 1 | **2.73** | 0.00144868 |
| v-myb myeloblastosis viral oncogene homolog (avian)-like 2 | **2.71** | 0.00989416 |
| KIT ligand | **2.70** | 0.00641955 |
| dual-specificity tyrosine-(Y)-phosphorylation regulated ki | **2.70** | 0.0234606 |
| intraflagellar transport 80 homolog (Chlamvdomonas) | **2.70** | 0.0247286 |
| transmembrane protein 48 | **2.69** | 0.00458248 |
| EBNA1 binding protein 2 | **2.69** | 0.00296292 |
| ZW10 interactor | **2.69** | 1.88E-05 |
| exonuclease 1 | **2.68** | 0.00739393 |
| transketolase (Wernicke-Korsakoff syndrome) | **2.68** | 1.92E-05 |
| somatostatin receptor 1 | **2.68** | 0.0144901 |
| isocitrate dehydrogenase 3 (NAD+) alpha | **2.67** | 0.00297129 |
| cytoskeleton associated protein 2 | **2.67** | 0.0030499 |
| minichromosome maintenance complex component 4 | **2.67** | 0.00342054 |
| inhibitor of DNA binding 1, dominant negative helix-loop-hel | **2.66** | 0.036485 |
| CDC28 protein kinase regulatory subunit 1B | **2.66** | 0.0145263 |
| keratin 18 | **2.66** | 8.40E-05 |
| CD97 molecule | **2.66** | 0.00994045 |
| chromosome 6 open reading frame 173 | **2.64** | 0.00222408 |
| BTB (POZ) domain containing 3 | **2.62** | 0.0166824 |
| deafness, autosomal dominant 5 | **2.62** | 0.00235481 |
| KIAA0286 protein | **2.62** | 0.00130563 |
| Fanconi anemia, complementation group D2 | **2.61** | 0.0281405 |
| polo-like kinase 4 (Drosophila) | **2.60** | 0.00209633 |
| ribonucleotide reductase M1 polypeptide | **2.60** | 0.000170076 |
| malic enzyme 1, NADP(+)-dependent, cytosolic | **2.59** | 0.0435444 |
| non-SMC condensin I complex, subunit H | **2.59** | 0.0216752 |
| S100 calcium binding protein A3 | **2.58** | 0.0324073 |
| ubiquitin-conjugating enzyme E2L 3 | **2.57** | 0.00343347 |
| BUB1 budding uninhibited by benzimidazoles 1 homolog beta | **2.56** | 0.0166047 |
| glycerol kinase | **2.55** | 2.66E-05 |
| TAF9B RNA polymerase II, TATA box binding protein (TBP)-as | **2.54** | 0.0170365 |
| TAF9B RNA polymerase II, TATA box binding protein (TBP)-as | **2.54** | 0.0170365 |
| histone cluster 1, H2bg | **2.52** | 0.000180822 |
| high-mobility group box 2 | **2.52** | 0.0196872 |
| NIMA (never in mitosis gene a)-related kinase 2 | **2.50** | 0.00289469 |
| proline rich 11 | **2.50** | 0.0357125 |
| myopalladin | **2.49** | 0.0255088 |
| brix domain containing 1 | **2.49** | 0.00471977 |
| cell division cycle associated 5 | **2.49** | 0.01021 |
| fucosidase, alpha-L- 2, plasma | **2.49** | 0.00540929 |
| cyclin-dependent kinase 2 | **2.49** | 0.00250724 |
| lamin B receptor | **2.49** | 0.000151784 |
| hypoxanthine phosphoribosyltransferase 1 (Lesch-Nyhan synd | **2.49** | 0.000634057 |
| tripartite motif-containing 25 | **2.47** | 0.0456344 |
| proteasome (prosome, macropain) subunit, beta type, 9 (lar | **2.46** | 0.0202595 |
| proteasome (prosome, macropain) subunit, beta type, 9 (lar | **2.46** | 0.0202595 |
| proteasome (prosome, macropain) subunit, beta type, 9 (lar | **2.46** | 0.0202595 |
| sphingomyelin synthase 2 | **2.46** | 0.0020701 |
| transmembrane protein 62 | **2.45** | 0.00761064 |
| glucose-6-phosphate dehydrogenase | **2.44** | 0.00278311 |
| PHD finger protein 1 | **2.44** | 0.010191 |
| retinoblastoma-like 1 (p107) | **2.44** | 0.00319946 |
| KIAA1524 | **2.43** | 0.0380688 |
| ST6 (alpha-N-acetyl-neuraminyl-2,3-beta-galactosyl-1, | **2.43** | 0.00830766 |
| cofilin 2 (muscle) | **2.43** | 0.0459235 |
| hypothetical protein LOC201725 | **2.42** | 0.000313319 |
| cell division cycle 25 homolog A (S. pombe) | **2.42** | 0.000341692 |
| breast cancer 1, early onset | **2.41** | 0.0180553 |
| transaldolase 1 | **2.41** | 0.00199537 |
| mRNA turnover 4 homolog (S. cerevisiae) | **2.41** | 0.00373104 |
| glucosaminyl (N-acetyl) transferase 1, core 2 (beta-1,6-N- | **2.41** | 0.0197148 |
| cysteine rich transmembrane BMP regulator 1 (chordin-like) | **2.41** | 0.0267286 |
| tissue factor pathway inhibitor (lipoprotein-associated | **2.40** | 0.0356227 |
| chromosome 16 open reading frame 59 | **2.40** | 0.00185191 |
| glycogenin 1 | **2**.**39** | 0.0224317 |
| transmembrane protein 154 | **2.39** | 0.0045589 |
| tubulointerstitial nephritis antigen-like 1 | **2**.**39** | 0.00510812 |
| CTP synthase | **2.38** | 8.80E-05 |
| phenylalanyl-tRNA synthetase, beta subunit | **2.38** | 0.000245973 |
| geminin, DNA replication inhibitor | **2.38** | 0.00167629 |
| lamin B1 | **2.37** | 0.0477748 |
| SPC24, NDC80 kinetochore complex component, homolog (S. ce | **2.36** | 0.00287227 |
| glutathione reductase | **2.36** | 0.00353875 |
| ribosomal protein L22-like 1 | **2.36** | 0.00335381 |
| fumarylacetoacetate hydrolase (fumarylacetoacetase) | **2.36** | 3.88E-05 |
| small nucleolar RNA, C | **2.35** | 0.0188991 |
| family with sequence similarity 64, member A | **2.35** | 0.0019785 |
| epithelial cell transforming sequence 2 oncogene | **2.35** | 0.000571152 |
| polymerase (DNA directed), epsilon 2 (p59 subunit) | **2.34** | 0.00479612 |
| glycerol kinase | **2.34** | 3.37E-06 |
| glutathione S-transferase M2 (muscle) | **2.33** | 0.0402076 |
| elongation factor, RNA polymerase II, 2 | **2.33** | 0.0130017 |
| thioredoxin | **2.33** | 0.009636 |
| polymerase (DNA directed), alpha 2 (70kD subunit) | **2.32** | 0.0033903 |
| breast cancer 2, early onset | **2.32** | 0.00586847 |
| CDC45 cell division cycle 45-like (S. cerevisiae) | **2.32** | 0.00735977 |
| H2A histone family, member Z | **2.32** | 0.0129697 |
| transporter 1, ATP-binding cassette, sub-family B (MDR | **2.31** | 0.0164234 |
| transporter 1, ATP-binding cassette, sub-family B (MDR | **2.31** | 0.0164234 |
| transporter 1, ATP-binding cassette, sub-family B (MDR | **2.31** | 0.0164234 |
| nucleolar complex associated 3 homolog (S. cerevisiae) | **2.30** | 0.000373346 |
| ATPase, Ca++ transporting, plasma membrane 4 | **2.30** | 0.023011 |
| minichromosome maintenance complex component 7 | **2.30** | 0.0457691 |
| TIMELESS interacting protein | **2.29** | 0.00771062 |
| von Hippel-Lindau binding protein 1 | **2.28** | 0.00329061 |
| ras-related C3 botulinum toxin substrate 2 (rho family, sma | **2.28** | 0.0292466 |
| thymopoietin | **2.28** | 0.0223176 |
| peptidylprolyl isomerase F (cyclophilin F) | **2.28** | 0.00093846 |
| activated leukocyte cell adhesion molecule | **2.27** | 0.00242163 |
| polvcomb group ring finger 5 | **2.27** | 0.000294142 |
| Ran GTPase activating protein 1 | **2.27** | 9.68E-05 |
| replication factor C (activator 1) 4, 37kDa | **2.26** | 0.00164152 |
| tubulin, beta 2C | **2.26** | 0.000346744 |
| minichromosome maintenance complex component 10 | **2.26** | 0.0037925 |
| H2B histone family, member S | **2.25** | 0.000885505 |
| gamma-glutamyl hydrolase (conjugase, folylpolygammaglutamyl | **2.25** | 0.0195219 |
| transcription termination factor, RNA polymerase II | **2.25** | 0.000393489 |
| polymerase (DNA directed), delta 2, regulatory subunit 50k | **2.25** | 0.0123823 |
| transporter 1, ATP-binding cassette, sub-family B (MDR | **2.25** | 0.00859077 |
| transporter 1, ATP-binding cassette, sub-family B (MDR | **2.25** | 0.00859077 |
| transporter 1, ATP-binding cassette, sub-family B (MDR | **2.25** | 0.00859077 |
| histone cluster 1, H2bf | **2.25** | 0.0124279 |
| eukaryotic translation initiation factor 1A, X-linked | **2.24** | 0.00330183 |
| phosphoglucomutase 2 | **2.24** | 0.00818204 |
| peroxisomal D3,D2-enoyl-CoA isomerase | **2.24** | 0.00148722 |
| interferon-induced protein with tetratricopeptide repeats | **2.24** | 0.0177928 |
| G-2 and S-phase expressed 1 | **2.23** | 0.0241887 |
| minichromosome maintenance complex component 2 | **2.23** | 0.0021347 |
| family with sequence similarity 72, member A | **2.23** | 0.00143248 |
| RMI1, RecQ mediated genome instability 1, homolog (S. cerev | **2.23** | 0.00294705 |
| FLJ20105 protein | **2.23** | 0.0127979 |
| multiple coagulation factor deficiency 2 | **2.22** | 0.0116892 |
| phytoceramidase, alkaline | **2.22** | 0.0157729 |
| coiled-coil domain containing 68 | **2.22** | 0.00227586 |
| dedicator of cytokinesis 11 | **2.21** | 0.00697577 |
| platelet-derived growth factor alpha polypeptide | **2.21** | 0.00176418 |
| N-acylsphingosine amidohydrolase (non-lysosomal cerami | **2.20** | 0.00728536 |
| S-phase kinase-associated protein 2 (p45) | **2.20** | 0.00230153 |
| polymerase (RNA) III (DNA directed) polypeptide G (32kD) | **2.20** | 0.0298794 |
| ADP-ribosylation factor-like 6 interacting protein 1 | **2.20** | 0.00139745 |
| histone cluster 1, H2bh | **2.19** | 0.0377748 |
| origin recognition complex, subunit 5-like (yeast) | **2.19** | 0.049697 |
| CDC28 protein kinase regulatory subunit 2 | **2.19** | 0.0128024 |
| histone cluster 1, H4c | **2.19** | 0.0112695 |
| hypothetical protein LOC729012 | **2.19** | 0.000446087 |
| DEAD (Asp-Glu-Ala-Asp) box polypeptide 39 | **2.19** | 0.000340561 |
| chromatin assembly factor 1, subunit B (p60) | **2.18** | 0.0119687 |
| MLF1 interacting protein | **2.18** | 0.0177203 |
| microtubule associated serine | **2.18** | 0.00536974 |
| MHC class I polypeptide-related sequence B | **2.18** | 0.0165406 |
| shugoshin-like 2 (S. pombe) | **2.18** | 0.000852557 |
| COP9 constitutive photomorphogenic homolog subunit 6 (Arab | **2.18** | 0.000793512 |
| methylenetetrahydrofolate dehydrogenase (NADP+ dependent) | **2.18** | 0.00119726 |
| chromosome 6 open reading frame 167 | **2.18** | 0.0011095 |
| pituitary tumor-transforming 1 | **2.17** | 0.0485166 |
| ribonuclease H2, subunit A | **2.17** | 0.00669936 |
| X-ray repair complementing defective repair in Chinese ham | **2.16** | 0.0369865 |
| membrane protein, palmitoylated 5 (MAGUK p55 subfamily memb | **2.16** | 0.00211873 |
| karyopherin alpha 2 (RAG cohort 1, importin alpha 1) | **2.16** | 0.000650645 |
| pleckstrin homology domain containing, family A (phosphoi | **2.15** | 0.0256434 |
| ribosomal protein L39-like | **2.15** | 0.00429384 |
| karvopherin alpha 2 (RAG cohort 1, importin alpha 1) | **2.15** | 0.000700649 |
| amyloid beta (A4) precursor protein-binding, family B, m | **2.15** | 0.00201004 |
| minichromosome maintenance complex component 3 | **2.14** | 0.0018389 |
| histone cluster 1, H2ai | **2.14** | 0.0129155 |
| chromosome 13 open reading frame 34 | **2.14** | 0.000702936 |
| RAD18 homolog (S. cerevisiae) | **2.14** | 0.0016685 |
| WD repeat and HMG-box DNA binding protein 1 | **2.13** | 0.0034833 |
| sulfide quinone reductase-like (yeast) | **2.13** | 0.0473641 |
| chromosome 16 open reading frame 63 | **2.12** | 0.000804179 |
| M-phase phosphoprotein 1 | **2.12** | 0.0271814 |
| minichromosome maintenance complex component 6 | **2.12** | 0.0161279 |
| homeobox A9 | **2.11** | 0.00520942 |
| fibroblast growth factor 9 (glia-activating factor) | **2.10** | 0.0475844 |
| cell division cycle 25 homolog C (S. pombe) | **2.10** | 0.0169914 |
| chromosome 9 open reading frame 64 | **2.10** | 0.0265979 |
| U2AF homology motif (UHM) kinase 1 | **2.09** | 0.0255167 |
| replication factor C (activator 1) 2, 40kDa | **2.09** | 0.00768959 |
| hypothetical protein LOC440894 | **2.09** | 0.0103358 |
| small nuclear ribonucleoprotein D1 polypeptide 16kDa | **2.09** | 0.0334665 |
| CSE1 chromosome segregation 1-like (yeast) | **2.09** | 0.0013662 |
| phosphatidylinositol glycan anchor biosynthesis, class W | **2.09** | 0.0151967 |
| centromere protein O | **2.09** | 0.00397056 |
| family with sequence similarity 20, member B | **2.09** | 0.00460031 |
| hypothetical protein FLJ40869 | **2.09** | 0.00444509 |
| guanine nucleotide binding protein (G protein), gamma 11 | **2.08** | 0.00140559 |
| calcyclin binding protein | **2.08** | 0.00524566 |
| ATP-binding cassette, sub-family E (OABP), member 1 | **2.08** | 0.00454751 |
| CD44 molecule (Indian blood group) | **2.08** | 0.000651436 |
| exosome component 8 | **2.08** | 0.00132017 |
| family with sequence similarity 102, member B | **2.08** | 0.025743 |
| histone cluster 2, H3d | **2.07** | 0.0102932 |
| family with sequence similarity 33, member A | **2.07** | 0.000318673 |
| Fanconi anemia, complementation group B | **2.07** | 0.000255109 |
| kinesin family member 22 | **2.07** | 0.0192406 |
| histone cluster 1, H2ai | **2.07** | 0.0161621 |
| vaccinia related kinase 1 | **2.06** | 0.0233182 |
| integrator complex subunit 7 | **2.06** | 0.000841371 |
| flap structure-specific endonuclease 1 | **2.06** | 0.006882 |
| hypothetical protein FLJ25416 | **2.06** | 0.000177531 |
| ecotropic viral integration site 2B | **2.06** | 0.0171408 |
| retinitis pigmentosa 2 (X-linked recessive) | **2.05** | 0.0264185 |
| centromere protein L | **2.05** | 0.000880856 |
| cofactor required for Sp1 transcriptional activation, subu | **2.04** | 0.00141809 |
| chromosome 20 open reading frame 121 | **2.04** | 0.0146323 |
| family with sequence similaritv 72, member A | **2.04** | 0.00162905 |
| family with sequence similarity 72, member A | **2.04** | 0.00165234 |
| eukaryotic translation initiation factor 1A, X-linked | **2.04** | 0.00520549 |
| elongation factor, RNA polymerase II, 2 | **2.03** | 0.0458007 |
| ATPase, Na+ | **2.03** | 0.0189108 |
| histone cluster 1, H3a | **2.03** | 0.0244273 |
| brix domain containing 1 | **2.03** | 0.00981178 |
| sushi domain containing 1 | **2.03** | 0.0258164 |
| ectonucleoside triphosphate diphosphohydrolase 6 (putativ | **2.03** | 0.00423628 |
| fructosamine 3 kinase | **2.03** | 0.00470972 |
| Bloom syndrome | **2.02** | 0.0209259 |
| tubulin, alpha 1c | **2.01** | 0.00862586 |
| E2F transcription factor 2 | **2.01** | 0.0496479 |
| exosome component 2 | **2.01** | 0.00649147 |
| kinesin family member 22 | **2.01** | 0.0242075 |
| LTV1 homolog (S. cerevisiae) | **2.01** | 0.00812652 |
| dihydrolipoamide S-acetyltransferase (E2 component of pyruv | **2.01** | 0.00179011 |
| v-ral simian leukemia viral oncogene homolog B (ras related | **2.01** | 0.012225 |
| ring finger and WD repeat domain 3 | **2.01** | 0.0013797 |
| annexin A1 | **2.01** | 0.0173578 |
| elaC homolog 2 (E. coli) | **2.00** | 0.00266504 |
| aldehyde dehydrogenase 9 family, member A1 | **2.00** | 0.00911609 |
| tubulin, alpha 4a | **2.00** | 0.0435427 |
| nuclear pore complex interacting protein | **-2.00** | 0.00111223 |
| oculomedin | **-2.01** | 0.00778869 |
| similar to PI-3-kinase-related kinase SMG-1 | **-2.01** | 0.0356628 |
| golgi autoantigen, golgin subfamily a-like pseudogene | **-2.01** | 0.00770626 |
| spectrin repeat containing, nuclear envelope 1 | **-2.01** | 0.00438469 |
| nuclear pore complex interacting protein | **-2.01** | 0.00117582 |
| sushi, nidogen and EGF-like domains 1 | **-2.01** | 0.00161129 |
| integrin, alpha V (vitronectin receptor, alpha polypeptide | **-2.02** | 0.00252702 |
| cyclin-dependent kinase inhibitor 2B (p15, inhibits CDK4) | **-2.04** | 0.0150268 |
| lysyl oxidase-like 4 | **-2.04** | 0.0120148 |
| nuclear pore complex interacting protein | **-2.04** | 0.000213956 |
| calcium | **-2.04** | 0.00657494 |
| calsyntenin 3 | **-2.04** | 0.00300887 |
| cell adhesion molecule 1 | **-2.05** | 0.0261129 |
| solute carrier family 22 (organic cation transporter), | **-2.05** | 0.0137275 |
| RUN and FYVE domain containing 3 | **-2.05** | 0.00387265 |
| glucosidase, alpha; acid (Pompe disease, glycogen storage di | **-2.05** | 0.000418401 |
| nuclear pore complex interacting protein | **-2.05** | 0.00988632 |
| proline-rich nuclear receptor coactivator 1 | **-2.06** | 0.0039587 |
| membrane metallo-endopeptidase | **-2.06** | 0.0152684 |
| PHD finger protein 21A | **-2.06** | 0.00980401 |
| Rho GTPase-activating protein | **-2.06** | 0.00705186 |
| homeobox B6 | **-2.06** | 0.00301714 |
| nuclear pore complex interacting protein | **-2.07** | 0.00032839 |
| phospholipase A2 receptor 1, 180kDa | **-2.07** | 0.00069343 |
| nuclear pore complex interacting protein | **-2.08** | 0.000352007 |
| slit homolog 3 (Drosophila) | **-2.08** | 0.02844 |
| nuclear pore complex interacting protein | **-2.09** | 0.000414309 |
| cyclin-dependent kinase 6 | **-2.09** | 0.0456892 |
| dynamin 1 | **-2.09** | 0.00139674 |
| jumonji, AT rich interactive domain 1B | **-2.09** | 0.00861002 |
| calcium binding and coiled-coil domain 1 | **-2.09** | 0.00370041 |
| insulin-like growth factor 1 receptor | **-2.09** | 0.00114467 |
| nuclear pore complex interacting protein | **-2.10** | 0.000377834 |
| CD82 molecule | **-2.10** | 0.0175517 |
| bromodomain adjacent to zinc finger domain, 2B | **-2.10** | 9.88E-05 |
| --- | **-2.10** | 0.00666187 |
| synaptotagmin XI | **-2.11** | 0.0129428 |
| KIAA1546 | **-2.11** | 0.000255634 |
| jun B proto-oncogene | **-2.12** | 0.0120169 |
| CXXC finger 6 | **-2.12** | 0.0277527 |
| nuclear pore complex interacting protein | **-2.14** | 0.00282604 |
| Cdon homolog (mouse) | **-2.15** | 0.0350357 |
| B-cell CLL | **-2.15** | 0.00343507 |
| nuclear pore complex interacting protein | **-2.15** | 0.00263888 |
| v-abl Abelson murine leukemia viral oncogene homolog 1 | **-2.16** | 0.0136688 |
| nuclear pore complex interacting protein | **-2.16** | 0.00583397 |
| FAT tumor suppressor homolog 1 (Drosophila) | **-2.18** | 0.0158766 |
| transformer-2 alpha | **-2.18** | 0.012256 |
| chimerin (chimaerin) 1 | **-2.18** | 0.0287031 |
| milk fat globule-EGF factor 8 protein | **-2.18** | 0.000987073 |
| vitamin D (1,25- dihydroxyvitamin D3) receptor | **-2.19** | 0.000192208 |
| neuroblastoma, suppression of tumorigenicity 1 | **-2.20** | 0.00090639 |
| jumonji domain containing 1A | **-2.20** | 0.0188513 |
| WNK lysine deficient protein kinase 1 | **-2.21** | 1.57E-05 |
| protocadherin beta 14 | **-2.21** | 0.0103892 |
| cortactin binding protein 2 | **-2.21** | 2.28E-05 |
| WW domain containing transcription regulator 1 | **-2.22** | 0.0379899 |
| cyclin L1 | **-2.22** | 0.00831474 |
| nuclear factor of activated T-cells, cytoplasmic, calcine | **-2.22** | 0.00786451 |
| pellino homolog 1 (Drosophila) | **-2.23** | 0.00939357 |
| golgi autoantigen, golgin subfamily a-like pseudogene | **-2.24** | 0.00603583 |
| chromosome 7 open reading frame 10 | **-2.26** | 0.00738442 |
| golgi autoantigen, golgin subfamily a-like pseudogene | **-2.27** | 0.00320764 |
| small Cajal body-specific RNA 17 | **-2.27** | 0.0301336 |
| latent transforming growth factor beta binding protein 2 | **-2.29** | 4.08E-05 |
| golgi autoantigen, golgin subfamily a, 8A | **-2.29** | 0.0111179 |
| inhibin, beta A (activin A, activin AB alpha polvpeptide) | **-2.29** | 0.00877271 |
| solute carrier family 41, member 2 | **-2.30** | 0.00453672 |
| forkhead box P1 | **-2.30** | 0.0463138 |
| matrix metallopeptidase 14 (membrane-inserted) | **-2.31** | 1.93E-05 |
| transcription factor 4 | **-2.31** | 0.0367869 |
| jun oncogene | **-2.32** | 7.21E-05 |
| neuroepithelial cell transforming gene 1 | **-2.33** | 0.0109689 |
| asporin | **-2.33** | 0.000659873 |
| v-fos FBJ murine osteosarcoma viral oncogene homolog | **-2.35** | 0.0138624 |
| ephrin-B2 | **-2.36** | 0.00611474 |
| WD repeat and SOCS box-containing 1 | **-2.36** | 0.0387851 |
| similar to dJ402H5.2 (novel protein similar to wo | **-2.36** | 0.00621503 |
| PX domain containing serine | **-2.38** | 0.000927628 |
| collagen, type VII, alpha 1 (epidermolysis bullosa, dystr | **-2.38** | 0.00109233 |
| AE binding protein 1 | **-2.39** | 0.000105628 |
| peroxidasin homolog (Drosophila) | **-2.40** | 0.00219049 |
| calcium channel, voltage-dependent, L type, alpha 1C sub | **-2.41** | 0.0189661 |
| Prader-Willi syndrome chromosome region 1 | **-2.45** | 0.0415526 |
| midline 1 (Opitz | **-2.45** | 0.00130803 |
| nuclear pore complex interacting protein | **-2.45** | 0.00354416 |
| chromosome 1 open reading frame 54 | **-2.47** | 0.0186089 |
| transmembrane protein 16A | **-2.48** | 0.0481085 |
| basic helix-loop-helix domain containing, class B, 2 | **-2.49** | 0.00270257 |
| nuclear pore complex interacting protein | **-2.50** | 0.00316496 |
| runt-related transcription factor 1 (acute myeloid leukemi | **-2.50** | 0.000607387 |
| zinc finger protein 292 | **-2.50** | 0.029832 |
| fibronectin leucine rich transmembrane protein 2 | **-2.51** | 0.0135122 |
| nuclear pore complex interacting protein | **-2.51** | 0.00283418 |
| potassium voltage-gated channel, subfamily G, member 1 | **-2.54** | 0.0244306 |
| interleukin 19 | **-2.54** | 0.0310328 |
| transforming growth factor, beta 3 | **-2.54** | 0.0287865 |
| dihydropyrimidinase-like 3 | **-2.55** | 0.0165203 |
| golgi autoantigen, golgin subfamily a, 8B | **-2.56** | 0.0121417 |
| hypothetical protein PRO2012 | **-2.57** | 0.00756704 |
| SATB homeobox 2 | **-2.57** | 0.039781 |
| t-complex 11 (mouse)-like 2 | **-2.57** | 0.0324227 |
| ring finger protein 122 | **-2.57** | 0.0236621 |
| chromosome 8 open reading frame 57 | **-2.59** | 0.00261522 |
| ADAM metallopeptidase with thrombospondin type 1 motif, | **-2.60** | 0.0113968 |
| sushi, von Willebrand factor type A, EGF and pentraxin dom | **-2.63** | 2.23E-05 |
| ST6 beta-galactosamide alpha-2,6-sialyltranferase 2 | **-2.64** | 0.0216987 |
| sortilin-related VPS10 domain containing receptor 2 | **-2.65** | 0.00936311 |
| protocadherin beta 9 | **-2.66** | 0.0285124 |
| chromosome 5 open reading frame 13 | **-2.67** | 0.00410172 |
| Enah | **-2.68** | 0.0077547 |
| pyridoxal-dependent decarboxylase domain containing 2 | **-2.69** | 0.00683647 |
| similar to nuclear pore complex interacting protein | **-2.70** | 0.0187322 |
| nuclear pore complex interacting protein | **-2.70** | 0.00368967 |
| transmembrane protein 119 | **-2.70** | 0.00801387 |
| chromosome 14 open reading frame 37 | **-2.70** | 0.0182453 |
| sushi-repeat-containing protein, X-linked 2 | **-2.71** | 0.0253856 |
| PDZ domain containing RING finger 3 | **-2.71** | 0.00931014 |
| collagen, type XII, alpha 1 | **-2.72** | 0.000204664 |
| matrix-remodelling associated 5 | **-2.72** | 0.000317637 |
| collagen, type V, alpha 1 | **-2.72** | 0.0166427 |
| dystrophin related protein 2 | **-2.72** | 0.0137557 |
| ATP-binding cassette, sub-family A (ABC1), member 1 | **-2.73** | 0.00131361 |
| trophinin | **-2.77** | 0.00298044 |
| cornichon homolog 3 (Drosophila) | **-2.78** | 0.0261738 |
| formin binding protein 1-like | **-2.78** | 0.00290401 |
| brain and acute leukemia, cytoplasmic | **-2.78** | 0.0476919 |
| protein tyrosine phosphatase, receptor type, U | **-2.80** | 0.0270428 |
| hypothetical protein MGC24103 | **-2.82** | 0.0346673 |
| interferon induced with helicase C domain 1 | **-2.83** | 0.0024839 |
| phospholipid transfer protein | **-2.84** | 0.00999206 |
| immediate early response 3 | **-2.87** | 0.0152127 |
| immediate early response 3 | **-2.87** | 0.0152127 |
| ADAM metallopeptidase domain 12 (meltrin alpha) | **-2.87** | 0.000870288 |
| synaptic vesicle glycoprotein 2A | **-2.88** | 0.00704212 |
| chromosome 9 open reading frame 3 | **-2.88** | 0.00410177 |
| thioredoxin interacting protein | **-2.90** | 0.0135494 |
| early growth response 1 | **-2.93** | 0.000425035 |
| small nucleolar RNA, C | **-2.94** | 0.00666866 |
| small nucleolar RNA, C | **-2.95** | 0.00765575 |
| immediate early response 3 | **-2.99** | 0.0167309 |
| low density lipoprotein-related protein 1 (alpha-2-macroglo | **-2.99** | 4.26E-05 |
| bicaudal C homolog 1 (Drosophila) | **-2.99** | 0.0347162 |
| homeobox B2 | **-3.03** | 0.00665994 |
| small nucleolar RNA, C | **-3.10** | 0.0274043 |
| small nucleolar RNA, C | **-3.10** | 0.0274043 |
| matrix metallopeptidase 2 (gelatinase A, 72kDa gelatinase, | **-3.13** | 5.59E-05 |
| KIAA1641 | **-3.14** | 0.00659194 |
| collagen, type VI, alpha 3 | **-3.14** | 2.09E-06 |
| homeobox A2 | **-3.15** | 0.0435423 |
| SH3 and PX domains 2B | **-3.15** | 0.0244357 |
| collagen, type VI, alpha 2 | **-3.16** | 0.0149554 |
| chromosome 9 open reading frame 3 | **-3.21** | 0.0233723 |
| small nucleolar RNA, C | **-3.24** | 0.0104491 |
| small nucleolar RNA, C | **-3.24** | 0.0104491 |
| - | **-3.27** | 0.00488845 |
| UDP-N-acetyl-alpha-D-galactosamine:polypeptide N-acetylga | **-3.35** | 0.00964109 |
| cholesterol 25-hydroxylase | **-3.38** | 0.0445558 |
| KIAA1641 | **-3.40** | 0.013175 |
| ring finger protein 144 | **-3.40** | 0.0135334 |
| versican | **-3.41** | 0.023885 |
| angiopoietin-like 2 | **-3.42** | 0.0245161 |
| KIAA1641 | **-3.44** | 0.0170531 |
| FBJ murine osteosarcoma viral oncogene homolog B | **-3.54** | 0.00025573 |
| similar to RIKEN cDNA 1110018M03 | **-3.59** | 0.00516476 |
| early growth response 2 (Krox-20 homolog, Drosophila) | **-3.62** | 0.00821813 |
| dachsous 1 (Drosophila) | **-3.63** | 0.00697244 |
| kinesin family member 26B | **-3.64** | 0.00363199 |
| distal-less homeobox 5 | **-3.66** | 0.000640157 |
| similar to Protein KIAA0220 | **-3.69** | 0.0302619 |
| insulin-like growth factor 1 receptor | **-3.71** | 3.42E-05 |
| protein tyrosine phosphatase, receptor type, N | **-3.77** | 0.0294569 |
| KIAA1641 | **-3.85** | 0.0191782 |
| sushi-repeat-containing protein, X-linked | **-3.85** | 0.00370941 |
| microfibrillar-associated protein 2 | **-3.91** | 0.0152901 |
| complement component 1, s subcomponent | **-3.97** | 0.0395863 |
| CD24 molecule | **-3.99** | 0.0340122 |
| homeobox B3 | **-4.02** | 0.0354368 |
| trichorhinophalangeal syndrome I | **-4.02** | 0.00557712 |
| Kallmann syndrome 1 sequence | **-4.04** | 0.000548703 |
| leucine rich repeat containing 17 | **-4.09** | 0.0263961 |
| plexin domain containing 2 | **-4.32** | 0.031799 |
| PTK7 protein tyrosine kinase 7 | **-4.42** | 0.000116114 |
| supervillin | **-4.43** | 0.0412717 |
| zinc finger protein 521 | **-4.58** | 0.00668815 |
| calbindin 2,29kDa (calretinin) | **-4.77** | 0.0290743 |
| ras homolog gene family, member J | **-4.79** | 0.00197982 |
| integrin, alpha 11 | **-4.80** | 0.000390317 |
| odz, odd Oz | **-5.05** | 0.00172671 |
| F-box protein 32 | **-5.52** | 0.0212957 |
| raftlin family member 2 | **-5.72** | 0.0260454 |
| clusterin | **-5.74** | 0.0303973 |
| neurotrimin | **-5.79** | 3.78E-06 |
| WNT1 inducible signaling pathway protein 1 | **-5.86** | 0.000672342 |
| insulin-like growth factor binding protein 5 | **-6.34** | 0.011614 |
| sulfatase 2 | **-6.34** | 5.88E-05 |
| microfibrillar-associated protein 4 | **-6.93** | 0.00155578 |
| junctional adhesion molecule 2 | **-7.07** | 0.0306758 |
| fibronectin type III domain containing 1 | **-7.29** | 0.0334696 |
| sarcoglycan, delta (35kDa dystrophin-associated glycoprotei | **-7.37** | 0.000881984 |
| hephaestin | **-7.53** | 0.0123141 |
| serpin peptidase inhibitor, clade F (alpha-2 antiplasmi | **-7.66** | 0.00362941 |
| cystatin SN | **-7.96** | 0.0496433 |
| hemicentin 1 | **-8.18** | 0.0461603 |
| tenascin C (hexabrachion) | **-8.32** | 8.26E-05 |
| biglycan | **-8.62** | 0.00161284 |
| transmembrane, prostate androgen induced RNA | **-11.20** | 0.000100935 |
| carboxypeptidase E | **-11.22** | 0.00738131 |

***Expression of cellular markers on PLX-C cells -*** the surface antigens expressed by PLX-C were examined using monoclonal antibodies. Results indicated that PLX-C were characterized by the positive markers: CD73, CD29 and CD105 and the negative markers: CD34, CD45, CD19, CD14 and HLA-DR (data not shown). The immune phenotype test specifications were set as: ≥ 90 % for all positive markers and ≤ 3 % for all negative markers.

Furthermore, as shown in Figures 2A-B, PLX-C cultures did not express endothelial markers as shown by negative staining for the two endothelial markers CD31 and KDR. However, PLX-C expression of a fibroblast-typical marker was evident (expression of D7-fib, Figure 2C).

***Immunogenecity and immunomodulatory properties of PLX-C cells-*** as PLX-C is comprised of adherent cells derived from placenta, it is expected to express HLA type I, which is expressed by all cells of the body and is known to induce an alloreactive immune response. HLA type II and other co-stimulatory molecules are typically expressed only on the surface of Antigen Presenting Cells (APCs).

In order to examine the immunogenicity of the obtained PLX-C cells, the expression of co-stimulatory molecules on the surface of these cell membranes were performed. FACS analysis demonstrated the absence of CD80, CD86 and CD40 on the PLX-C cell membranes (Figures 3A-C). Moreover, PLX-C expressed low levels HLA class I as detected by staining for HLA A/B/C (Figure 3D). The expression of stimulatory and co-stimulatory molecules was similar to bone marrow (BM) derived adherent cells (as shown in Figures 3A-D).

To further investigate the immunogenecity as well as the immunomodulation properties of PLX-C cells, Mix Lymphocyte Reaction (MLR) tests were performed. As shown in Figure 4A-B, PLX-C cells both escape allorecognition, and reduce T cell response, as measured by Thymidine incorporation. Furthermore, the reduction in lymphocytes proliferation (evaluated by CPM measurement) was higher as the number of PLX-C cells increased (in a dose dependent manner). PLX-C also reduced lymphocyte proliferation following mitogenic stimuli, such as Concavalin A (Con A, Figure 4B) and Phytohemagglutinin (PHA), and non-specific stimulation by anti-CD3, anti-CD28 (data not shown).

In order to investigate the mechanism of action by which PLX-C immunomodulate lymphocyte proliferation, and to see if this action is mediated via cell to cell interaction or cytokines secretion, PB derived Mononuclear cells (MNCs) were stimulated by PHA using the transwell method (which prevents cell to cell contact but enables the diffusion of cytokines between the two compartments). Results showed that the inhibition of proliferation maintained even when cell to cell contact was inhibited (data not shown).

***Cytokines secretion -*** as depicted hereinabove, PLX-C reduce the proliferation rate of lymphocytes, probably through soluble factors. Further investigation of the cytokines secreted by lymphocytes in response to PLX-C was performed to elucidate the mechanism of action of PLX-C. As depicted in Figures 5A-B, culturing of mononuclear cells with PLX-C slightly reduces the secretion of the pro-inflammatory cytokine INFγ and dramatically reduces the secretion of TNFα (even in the presence of low amounts of PLX-C). In addition, following lipopolysaccharide (LPS) stimulation, PB derived MNCs secretion of IL-10 increased in the presence of PLX-C, while the secretion level of TNFα decreased, in a dose dependent manner (Figure 5C).

### EXAMPLE 2

### RELEASE CRITERIA OF PLX-C FOR THE TREATMENT OF PERIPHERAL ARTERIAL DISEASE

Based on the above experimental data, criteria for selecting PLX-C batches of cells, which present a combination of characteristics that makes them most suitable for PAD treatment, were defined. These criteria are summarized in Table 3, below.

**Table 3: Criteria for selection of PLX-C cells for the treatment of PAD**

| **Test** | **Method** | **Specification** |
|---|---|---|
| Mycoplasma | JP XIV and EP 2.6.7 (Agar-Broth Culture Method) | Negative |
| Sterility | USP, <71> and EP 2.6.1 (Immersion) | No growth |
| Endotoxin | LAL Gel-Clot Technique | ≤ 10 EU/ ml |
| Viability | Trypan Blue | ≤ 70% |
| Yield | Trypan Blue | ≥ 60% |
| Identity/Purity | Flow Cytometer | ≥90% positive markers |
| Immune phenotype | | ≤3% negative markers |
| *In vitro* potency assay | PHA | TBD |
| | | Homogenous, opaque (not cloudy), |
| Appearance | Visual Inspection | off-white to yellowish in color, without foreign particles |

### Analytical Procedures

***Appearance:*** The cell suspension, placed in a 50 ml clear tube is visualized holding the tube against a white background in bright light; the cells are inspected for homogeneity, color and structure.

***Potency assay:*** In order to assess the specific ability of PLX-C to induce the expected therapeutic effect, experiments were performed in order to find a correlation between *in- vitro* characterizations and *in- vivo* activity.

Critical Limb Ischemia in PAD patients may occur as a result of atherosclerosis or inflammatory processes. Pre-Clinical data demonstrated that administration of PLX-PAD to ischemic mice, increased blood flow and reduced endothelial inflammation and oxidative stress.

Inflammation reduction and pro-angiogenesis properties of the cells are being evaluated in order to explain the in-vivo improvement following PLX-C administration. Inflammation is the process by which the body's white blood cells are activated in response to stimulation. PLX-C are characterized by their ability to suppress activated white blood cells. This feature is thought to be attributed to the in vivo antiinflammatory effect of PLX-C and may play a role in affecting progress of PAD. Inflammatory phenomena at sites of atherosclerotic plaques are increasingly thought to be major determinants of the progression and clinical outcome of atherosclerotic disease (Fiotti, Giansante et al. 1999). Thus, the ability of PLX-C to suppress proliferation of Peripheral Blood (PB) derived MNCs stimulated by PHA is under evaluation to serve as the potency assay.

In this assay, 2x10⁵ peripheral blood (PB) derived MNC are stimulated with 10ug PHA/ml Phytohemagglutinin (PHA). PLX cells are co-cultured with the MNCs (1:10 and 1:5). Three replicates of each group are seeded in a 96-well plate. Cells are cultured in RPMI 1640 medium containing 20% FBS. Plates are pulsed with 1µC ³H-thymidine during the last 18hr of 5-days culturing. Cells are harvested over a fiberglass filter and thymidine incorporation is quantified with a scintillation counter. Inhibition of lymphocytes proliferation should be 30±20% (lymphocytes proliferation of 70±20%. Based on the results PLX-C reduced lymphocyte proliferation, although the effect was more robust on Day 5, results of Day 3 are more sensitive to minor variations between the various samples.

***Immuno phenotype:*** In order to characterize the surface antigens expressed by PLX-PAD, the cells are stained with monoclonal antibodies for the following ASC characterized positive markers: CD73, CD29, CD105 and negative markers: CD34, CD45, CD14 and HLA-DR.

The immune phenotype tests are performed using the FC 500 Flow Cytometry System (Beckman Coulter) with CXP analysis Software. This Flow Cytometer conducts 5-color analysis from a single laser excitation.

Prior to each working day with the FACS system, a flow check is run using standard beads, to ensure that the system is calibrated.

Prior to performing each immune phenotypic test, international traceable standard solutions (CD Chex plus and CD chex cd34) are run to verify that all reagent and performance are intact.

In addition, before each test is performed and after adding the appropriate Antibodies, the method is qualified for reproducibility.

***Endotoxin:*** Endotoxin testing, using the Limulus Amebocyte Lysate (LAL) in a Gel-Clot Technique is performed by Hy-Labs (Rehovot, Israel) according to current USP 31, <85> and EP 2.6.14 procedures for biological endotoxin testing.

Inhibition / Enhancement (I/E) tests are performed on three product batches in order to determine that the LAL Gel-Clot method is working correctly.

Assay sensitivity is 0.03 EU/ml.

***Sterility:*** Sterility testing for the final product is performed according to current EP 2.6.1, current USP 30 <71> and 21 CFR 610.12.

The test method; Direct transfer/inoculation uses the set of challenges described under Growth Promotion Test of Aerobes, Anaerobes and Fungi (GPT), including Gram positive and Gram negative bacteria.

| | | |
|---|---|---|
| *For TSB Growth Promotion and Bacteriostasis and Fungistasis Tests* | *Bacillus subtilis* | *ATCC 6633* |
| | *Candida albicans* | *ATCC 10231* |
| | *Aspergillus niger* | *ATCC 16404* |
| *For FTM Growth Promotion and Bacteriostasis Tests* | *Staphylococcus aureus* | *ATCC 6538* |
| | *Clostridium sporogenes* | *ATCC 11437* |
| | *Pseudomonas aeruginosa* | *ATCC 9027* |

Incubation containers are kept in a 20-25 °C for TSB and 30-35 °C for FTM, for 14 days. Each test container is observed daily. Items that pass the sterility test are those where no growth of microorganisms was detected in any of the containers over a period of 14 days incubation.

***Mycoplasma:*** A Mycoplasma PCR based test is performed in-process in order to release the placenta from the quarantine area and initiate the PLX manufacturing phase, the PCR method was selected due to the short period required for obtaining results. At 3D Harvest a sample for mycoplasma testing (Agar-Broth Culture Method; JP XIV and EP 2.6.7) is collected and used for product release, this assay is performed according to JP and EP requirements.

***Cell counting and Viability:*** Both the 2DCS and PLX-C cells are counted by the Cedex HiRes which is an automated cell analyzer. Determination of percentage of cell viability is performed manually according to a Trypan Blue staining. Trypan Blue is a vital stain used to distinguish viable from nonviable cells. Viable cells exclude the dye, while nonviable cells absorb the dye and appear blue. Therefore, the percentage of viable cells is the number of viable cells divided by the total number of cells (dead plus viable cells).

### EXAMPLE 3

### RELEASE CRITERIA OF SUBJECTS FOR THE TREATMENT OF PERIPHERAL ARTERIAL DISEASE BY PLX-C CELLS

Patients with peripheral arterial disease (PAD) are treated with allogeneic placental PLX-C cells of the present teachings to determine if injections of PLX-C can be used safely and efficaciously to treat critical limb ischemia.

Two phase I studies are scheduled. These open-label, dose-escalation studies will be performed in parallel in the EU and US. The studies design is similar; however not identical, the follow up period and dose escalation vary. The clinical follow up period for both studies will last three months following treatment, however, in Germany the subjects will be further monitored for tumorigenesis up to 24 months in comparison to 12 months follow-up in the US for delayed adverse events. Furthermore, the intramuscular administration of PLX-C to the affected leg in the US, will be injected in one session for the low dose, and in two sessions (recurrent two administrations, two weeks apart) for the higher dose, whereas, in Germany the higher dose will be administered in a single session using an elevated volume per injection of PLX-PAD.
- Dosing schedule; Germany:
   ∘ Dosage group 1 (low dose group): 175x10⁶
   ∘ Dosage group 2 (intermediate dose group) 315x10⁶
   ∘ Dosage group 3 (high dose group): 595x10⁶
- Dosing schedule; US:
   ∘ Dosage group 1 (low dose group) 280x10⁶ cells
   ∘ Dosage group 2 (high dose group) 280x10⁶ *2(=560x10⁶) cells

### Release criteria for inclusion of the subjects in the study

The patients to be included in the study need to fulfill the following criteria:
1. Adult male or female subjects between 40 to 81 years of age at the time of screening visit.
2. Diagnosis of chronic critical limb ischemia defined as persistent, recurring ischemic rest pain for at least two (2) weeks, and/or ulceration or gangrene of the foot or toe, with ABI ≤ 0.6. (ABI- Ankle-Brachial Index)
   a. If the subject has an ABI ≥ 1.3 or the ABI cannot be calculated, then TBI ≤ 0.4.( TBI- Toe Brachial Index)
   b. If the diagnosis of CLI is unable to be made via TBI and/or ABI, the investigator may make the diagnosis via duplex scanning. If duplex scanning is unavailable, the investigator may use magnetic resonance angiography or computed tomographic angiography.
3. Diagnosis of CLI, Rutherford category 4-5.
4. Non candidate for revascularization or endovascular intervention based on unfavorable vascular anatomy or significant co-morbid medical conditions as confirmed by vascular study (e.g., angiogram, MRA) obtained within 3 months prior screening visit and signed approval of vascular surgeon. The decision to classify the subject as a non-candidate will be made by the investigator and confirmed by an independent third party vascular surgeon who is not participating in the study.
5. In the opinion of the investigator, major amputation is not anticipated over a period of three (3) months.
6. Normal organ and marrow function as defined:
   - Leukocytes ≥3,000/µL
   - Absolute neutrophil count ≥1,500/µL
   - Platelets ≥140,000/µL
   - AST (SGOT)/ALT (SGPT) ≤2.5 X institutional standards range
   - Creatinine clearance > 40 cc/min
   - INR≤ 1.5
   - Hematocrit > 36%
7. Subject must be on maximal medical therapy (e.g., anti-platelet drug, lipid lowering medication as needed, anti-hypertensive as needed, smoking cessation as appropriate, etc.).
8. Those diabetic subjects who are on optimal diabetes medication, with an HbA1c < 8%.
9. Signed written informed consent to participate in the study by subject

### Release criteria for exclusion of the subjects in the study

The patients to be excluded from the study include the following criteria:
1. Uncontrolled hypertension (defined as diastolic blood pressure > 110 mmHg or systolic blood pressure > 180 mmHg during screening).
2. Wounds with severity greater than Grade 2 on the Wagner Scale.
3. Previous amputation of the talus, or above in the target limb.
4. Successful lower extremity revascularization surgery (bypass or angioplasty of the leg to be treated) within 3 months prior to randomization.
5. Life-threatening ventricular arrhythmia - except if an ICD (Implantable Cardioverter Defibrillator) is implanted - or unstable angina - characterized by increasingly frequent episodes with modest exertion or at rest, worsening severity, and prolonged.
6. ST segment elevation myocardial infarction and/or TIA/CVA (Transient Ischemic Attacks / CerebroVascular Accident) within six (6) months prior to enrollment. Patients with severe congestive heart failure symptoms (i.e. NYHA Stage IV).
7. Infection of the involved extremity(ies) manifest by fever, purulence and severe cellulites.
8. Subject requiring uninterruptible anticoagulation or that cannot be stopped for 72 hours prior to investigational treatment.
9. Blood clotting disorder not caused by medication.
10. Subject has malignancy undergoing treatment including chemotherapy, radiotherapy or immunotherapy.
11. Diagnosis of end stage renal disease requiring dialysis.
12. Pregnant or breast-feeding women or women of childbearing potential not protected by an effective contraceptive method of birth control.
13. Known allergies to protein products (horse or bovine serum, or porcine trypsin) used in the cell production process.
14. Body Mass Index (BMI) of 40 Kg/m² or greater.
15. HIV, syphilis, positive at time of screening.
16. Active Hepatitis B, or Hepatitis C infection at the time of screening.
17. Positive for Tuberculosis (TB-specific T cell response).
18. Subject undergoing hyperbaric oxygen treatment within four (4) weeks of inclusion and/or required throughout the trial.
19. Concomitant wound treatments that include growth factor therapy or tissue engineered products within 8 weeks prior to enrollment,.
20. In the opinion of the investigator, the patient is unsuitable for cellular therapy.
21. Subject receiving systemic or direct target limb injection of antiangiogenic drugs.
22. Subject is currently enrolled in, or has not yet completed a period of at least 30 days since ending other investigational device or drug trial(s).
23. Subjects unwilling or unable to comply with the study procedures.

### Study Population

Enrollment into the study is limited to patients aged 18 to 81. Patients are selected by trial investigator based on the inclusion/exclusion criteria provided hereinabove.

Screening that can be accomplished without laboratory results (see inclusion/exclusion criteria, hereinabove), will be completed before enrollment of the patient. Once initial screening is complete, the patient should complete a consent form which should be reviewed with the patient and signed. After receipt of a signed consent form, laboratory tests (see hereinbelow) will be accomplished in order to complete the screening process. Patients who qualify after screening laboratory results have been reviewed, can be formally enrolled into the trial.

### Study Design Plan

Up to 30 patients with critical limb ischemia (CLI) will be enrolled into the two phase I studies

### Protocol synopsis for the US study:

### Protocol Synopsis

TITLE: Safety of Intramuscular (IM) Injection of Allogeneic PLX-PAD Cells for the Treatment of Critical Limb Ischemia.

### PROTOCOL NUMBER: PLX-PAD-1202-2

**INVESTIGATIONAL PRODUCT:** PLX-PAD, adherent stromal cell (ASC) based product isolated from human placenta

### PHASE: I

**INDICATION:** Critical limb ischemia (CLI), Rutherford category 4-5
**STUDY OBJECTIVES:** The objective of this study is to assess the safety of PLX-PAD Intra-muscular injection for the treatment of CLI patients.
**POPULATION:** This study will be conducted in approximately 12 subjects aged 40 to 81 years diagnosed with critical limb ischemia.

### STUDY DESIGN, DURATION and PROCEDURES:

This will be a Phase I, open-label, concurrent controlled, dose-escalation study comprising two (2) dosage groups with at most six (6) subjects in each dosage group.

Subjects will undergo similar study procedures and evaluations; however each dosage group will receive one of two targeted dose of placental-derived ASC's (PLX-PAD).

The treatment with the higher PLX-PAD dose will be divided into two sessions, separated two weeks apart.

The study will comprise four (4) periods:
1. Screening Period of up to four (4) weeks
2. Treatment of placental derived ASC's (PLX-PAD) followed by six (6) hours of monitoring
3. Clinical Follow up period for three (3) months
4. Long term follow up period for additional 9 months and termination visit

### Protocol Synopsis

### Period I (Screening Period): -4 Weeks to -Day -1

The screening visit (Visit 1) will include signing the informed consent, Inclusion/Exclusion assessment, diagnosis confirmation, laboratory tests, medical history, physical examination, ECG, and concomitant current medication documentation.

### Period II (PLX-PAD treatment): Day 0

Following the Screening Period, subjects will receive one of the targeted doses of PLX-PAD via IM injection. On the treatment day, prior to the IM injections, vital signs, laboratory tests, ECG, hemodynamic measurements (ABI, TBI & tcPO2) and wound assessment will be performed as well as King's College VascuQoL Questionnaire and pain assessment using the VAS scale.

PLX-PAD will be administered via thirty (30) intramuscular injections delivered to the affected leg for the low dose, for the higher dose, PLX-PAD will be administered twice (two sessions, two weeks apart), while at each session thirty (30) intramuscular injections are delivered to the affected leg.

A map of the injection site will be marked prior to injection, keeping a 1-2cm distance between each injection site.

Both legs will be photographed by a digital camera.

Subjects will then be monitored for a 6 hr. observation. Prior to release from observation, vital signs, blood tests and ECG will be performed.

### Period III (Follow up): Day 1 to Month 3

During the follow up period subjects will visit the Medical Center at Day 1, week 1 & 2, and at month 1 & 2 [total five (5) visits] for follow-up by a clinical investigator.

During the follow up visits blood samples will be withdrawn for biochemistry, hematology, immunology, hemodynamic measurements and wound assessment will be performed as well as King's College VascuQoL Questionnaire and pain assessment using the VAS scale.

At all visits both limbs will be photographed by a digital camera.

### Period IV (Follow up): Month 3 to Month 12

During the long term follow up period subjects will visit the Medical Center at month 6 and 12 for follow-up by a clinical investigator. ECG and blood sampling, hemodynamic measurements and wound assessment will be performed.

Termination visit and end of follow up period will be performed 12 months following PLX-PAD treatment.

### DOSAGE FORMS AND ROUTE OF ADMINISTRATION:

Three patients will be treated with the lowest dose of PLX-PAD. If no significant adverse reaction occurs, this dose will be given to another three patients.

If the low dose is well tolerated another three patients will receive the higher dose. If this is well tolerated another three patients will receive this dose. The higher dose is administrated in two sessions; two weeks a apart
- Dosage group 1 (low dose group) 280x10⁶ cells
- Dosage group 2 (high dose group) 280x10⁶ *2(=560x10⁶) cells

The first subject in each dose group will receive a single dose of the active PLX-PAD and followed for 24 hr prior to treating the next subject. Further dose administration will be performed sequentially.

The decision to escalate to the higher dose group will be made after the subjects at the low dose level have completed four (4) weeks of follow up.

If a significant adverse reaction occurs at any dose DSMB will make the decision whether the trial is to be stopped or further evaluations are required prior to the continuation of patient enrollment.

### Protocol Synopsis

### INCLUSION AND EXCLUSION CRITERIA

### Inclusion Criteria:

1. Adult male or female subjects between 40 to 81 years of age at the time of screening visit.
2. Diagnosis of chronic critical limb ischemia defined as persistent, recurring ischemic rest pain for at least two (2) weeks, and/or ulceration or gangrene of the foot or toe, with ABI ≤ 0.6.
   a. If the subject has an ABI ≥ 1.3 or the ABI cannot be calculated, then TBI ≤ 0.4.
   b. If the diagnosis of CLI is unable to be made via TBI and/or ABI, the investigator may make the diagnosis via duplex scanning. If duplex scanning is unavailable, the investigator may use magnetic resonance angiography or computed tomographic angiography.
3. Diagnosis of CLI, Rutherford category 4-5
4. Non candidate for revascularization or endovascular intervention based on unfavorable vascular anatomy or significant co-morbid medical conditions as confirmed by vascular study (e.g., angiogram, MRA) obtained within 3 months prior screening visit and signed approval of vascular surgeon. The decision to classify the subject as a non-candidate will be made by the investigator and confirmed by an independent third party vascular surgeon who is not participating in the study.
5. In the opinion of the investigator, major amputation is not anticipated over a period of three (3) months.
6. Normal organ and marrow function as defined:
   - Leukocytes ≥3,000/µL
   - Absolute neutrophil count ≥1,500/µL
   - Platelets ≥140,000/µL
   - AST (SGOT)/ALT (SGPT) ≤2.5 X institutional standards range
   - Creatinine clearance > 40 cc/min
   - INR≤ 1.5
   - Hematocrit > 36%
7. Subject must be on maximal medical therapy (e.g., anti-platelet drug, lipid lowering medication as needed, anti-hypertensive as needed, smoking cessation as appropriate, etc.).
8. Those diabetic subjects who are on optimal diabetes medication, with an HbA1c < 8%.
9. Signed written informed consent to participate in the study by subject.

### Exclusion Criteria:

1. Uncontrolled hypertension (defined as diastolic blood pressure > 110 mmHg or systolic blood pressure > 180 mmHg during screening).
2. Wounds with severity greater than Grade 2 on the Wagner Scale.
3. Previous amputation of the talus, or above in the target limb.
4. Successful lower extremity revascularization surgery (bypass or angioplasty of the leg to be treated) within 3 months prior to randomization.
5. Life-threatening ventricular arrhythmia - except if an ICD is implanted - or unstable angina - characterized by increasingly frequent episodes with modest exertion or at rest, worsening severity, and prolonged.
6. ST segment elevation myocardial infarction and/or TIA/CVA within six (6) months prior to

### Protocol Synopsis

enrollment. Patients with severe congestive heart failure symptoms (i.e. NYHA Stage IV).
7. Infection of the involved extremity(ies) manifest by fever, purulence and severe cellulites.
8. Subject requiring uninterruptible anticoagulation or that cannot be stopped for 72 hours prior to investigational treatment.
9. Blood clotting disorder not caused by medication.
10. Subject has malignancy undergoing treatment including chemotherapy, radiotherapy or immunotherapy.
11. Diagnosis of end stage renal disease requiring dialysis.
12. Pregnant or breast-feeding women or women of childbearing potential not protected by an effective contraceptive method of birth control.
13. Known allergies to protein products (horse or bovine serum, or porcine trypsin) used in the cell production process.
14. Body Mass Index (BMI) of 40 Kg/m² or greater.
15. HIV, syphilis, positive at time of screening.
16. Active Hepatitis B, or Hepatitis C infection at the time of screening.
17. Positive for Tuberculosis (TB-specific T cell response).
18. Subject undergoing hyperbaric oxygen treatment within four (4) weeks of inclusion and/or required throughout the trial.
19. Concomitant wound treatments that include growth factor therapy or tissue engineered products within 8 weeks prior to enrollment,.
20. In the opinion of the investigator, the patient is unsuitable for cellular therapy.
21. Subject receiving systemic or direct target limb injection of antiangiogenic drugs.
22. Subject is currently enrolled in, or has not yet completed a period of at least 30 days since ending other investigational device or drug trial(s).
23. Subjects unwilling or unable to comply with the study procedures.

### SAFETY VARIABLES:

### Safety Endpoints

1. Amputation incidence at three (3) months post treatment
2. Death incidence at three (3) months post treatment
3. Rehospitalization incidence at three (3) months post treatment
4. Adverse events
5. Immunological reaction

### Protocol Synopsis

### STATISTICAL METHODS:

**Descriptive statistics:** Descriptive statistics will be used to summarize the endpoints and baseline characteristics. In this analysis all available data will be presented with no imputation for any missing data. Subjects will contribute to the data available up to the point of withdrawal, study completion or death. The descriptive statistics such as means, median, standard deviation minimum and maximum will be used to summarize continuous variables. Box-plots will be generated for scanning outcome distributions and initial detection of outlier observations. Dichotomous variables will be presented in count and percentages.

**Dose-Response Modeling:** For the key safety outcome variable a logistic model will be fitted to the observed events if there sufficient data available. In this case the dose-response curve along with a 95% confidence interval will be drawn. This curve serves to obtain the Maximum Tolerated Dose (MTD) by interpolation or extrapolation. This analysis will be performed if all dose levels were given.

**Endpoints analysis:** A dedicated analysis will be generated for each of the study outcomes over time.

**SAMPLE SIZE DETERMINATION:** The sample size determination is not based on a formal analysis but on clinical experience. However, based on a sample size of up to 12 subjects (as intended) the probability to detect at least one adverse event is equal to 0.794, assuming that the incidence of an adverse event is given by 0.1. This probability is obtained using the binomial distribution.

### Protocol synopsis for the EU study:

### Protocol Synopsis

### TITLE: Safety of Intramuscular (IM) Injection of Allogeneic PLX-PAD Cells for the Treatment of Critical Limb Ischemia.

### PROTOCOL NUMBER: PLX-PAD-1202

**INVESTIGATIONAL PRODUCT:** PLX-PAD, adherent stromal cell (ASC) based product isolated from human placenta

### PHASE: I

**INDICATION:** Critical limb ischemia (CLI), Rutherford category 4-5
**STUDY OBJECTIVES:** The objective of this study is to assess safety of PLX-PAD single dose, Intra-muscular injection for the treatment of CLI patients.
**POPULATION:** This study will be conducted in approximately 18 subjects aged 40 to 81 years diagnosed with critical limb ischemia.

### STUDY DESIGN, DURATION and PROCEDURES:

This will be a Phase I, open-label, concurrent controlled, and dose-escalation study comprising three (3) dosage groups with at most six (6) subjects in each dosage group. This is a variant of the "3+3" design used in dose escalation. There are three doses with an intended cohort size of three or six patients per dose.

Subjects will undergo similar study procedures and evaluations; however each dosage group will receive one of three targeted dose of placental derived ASC's (PLX-PAD).

The study will comprise four (4) periods:
1. Screening Period of up to four (4) weeks
2. Single dose treatment of placental derived ASC's (PLX-PAD) followed by a minimum of five (5) days in clinic admission for observation
3. Follow up period for three (3) months
4. Long term follow up period for delayed adverse events for an additional 21 months and termination visit

### Protocol Synopsis

### Period I (Screening Period): -4 Weeks to -Day -1

The screening visit (Visit 1) will include signing the informed consent, inclusion/exclusion assessment, diagnosis confirmation, laboratory tests, medical history, physical examination, legs and abdomen sonography, ECG, and concomitant current medication documentation. An updated angiogram is required and should be performed in the last three weeks.

### Period II (Single dose treatment): Day 0

Following the Screening Period, subjects will receive one of the targeted doses of PLX-PAD IM injection under short anesthesia. On the treatment day, prior to IM injection vital signs, laboratory tests, ECG, hemodynamic measurements (ABI, TBI & tcPO2) and wound assessment will be performed as well as King's College VascuQoL Questionnaire and pain assessment using the VAS scale.

A map of the injection site will be marked prior to injection, keeping a 1-2cm distance between each injection site. The injections should follow the ischemic path based on an angiogram.

Prior to IM injection of PLX-PAD prophylactic antibiotic and an analgesic agent will be administered. PLX-PAD will be administered via 50 intramuscular injections delivered to effected leg.

Both legs will be Photographed by a digital camera.

Subject will be admitted for a minimum of five (5) days for in clinic observation. Prior to release, blood tests and ECG will be performed.

### Period III (Follow up): Day 1 to Month 3

During the follow up period subjects will visit the Medical Center at month 1, 2 & 3 [total three (3) visits] for follow-up by a clinical investigator.

During the follow up visits blood samples will be withdrawn for biochemistry, hematology, immunology, hemodynamic measurements and wound assessment will be performed as well as King's College VascuQoL Questionnaire and pain assessment using the VAS scale.

At all visits both Limbs will be photographed by a digital camera.

### Period IV (long term Follow up): Month 3 to month 24

During the long term follow up period subjects will visit the Medical Center at month 6, 12 and 18 for follow-up by a clinical investigator. ECG and blood sampling, hemodynamic measurements and wound assessment will be performed as well as King's College VascuQoL Questionnaire and pain assessment using the VAS scale. Legs and abdomen sonography will be performed at month 6, 12 and 24.

### DOSAGE FORMS AND ROUTE OF ADMINISTRATION:

This study will apply a variant of the 3+3 design for dose escalation.

Three patients will be treated with lowest dose of PLX-PAD. If no significant adverse reaction (also see section 6.1.4.) occurs the study will proceed to the next dose level. The first assessment of the significance is performed by the investigator.

The intermediate dose level will be given to three patients. Even if no significant adverse reaction occurs, this dose will be given to another three patients. That is, if the intermediate dose is reached a maximum of six patients will receive this dose.

If the intermediate dose is well tolerated another three patients will receive the highest dose. If this is well tolerated another three patients will receive this dose.
- Dosage group 1 (low dose group): 175x10⁶
- Dosage group 2 (intermediate dose group) 315x10⁶
- Dosage group 3 (high dose group): 595x10⁶

The first subject in each dose group will receive a single dose of the active PLX-PAD and followed for 36 hr prior treating the next subject. Further dose administration will be performed sequentially.

### Protocol Synopsis

The decision to escalate to a higher dose group will be taken after the subjects at the respective dose level have completed four (4) weeks of follow up, following single dose treatment, including safety evaluation.

An interim analysis will be performed following the completion of two (2) month follow up for at most six (6) subjects at the intermediate dose group.

If a significant adverse reaction occurs at any dose DSMB will take the decision whether the trail is to be stopped or further evaluation will be performed prior continuation of patient enrolment.

### INCLUSION AND EXCLUSION CRITERIA

### Inclusion Criteria:

1. Adult male or female subjects between 40 to 81 years of age at the time of screening visit
2. Diagnosis of critical limb ischemia defined as persistent, recurring ischemic rest pain for at least two (2) weeks, and/or ulceration or gangrene of the foot or toe, with ABI ≤ 0.4 or/and TBI ≤ 0.4 or transcutaneous partial pressure of oxygen ≤ 30 mmHg pO2 at the foot.
3. Rutherford category 4-5
4. No acceptable options for re-vascularisation as confirmed by angiographic imaging results or by color flow duplex ultrasound obtained within 6 months prior screening visit and signed approval of vascular surgeon.
5. In the opinion of the investigator, major amputation is not anticipated over a period of three (3) months.
6. Normal organ and marrow function as defined:
   Leucocytes ≥ 3,000/µL
   Absolute neutrophil count ≥1,500/µL
   Platelets ≥ 140,000/µL
   AST (SGOT)/ALT (SGPT) ≤ 2.5 X institutional standards range Creatinine clearance ≥ 30 cc/min
   INR ≤ 1.5
7. Negative Pregnancy test
8. Highly effective contraception in women (defined as pearl index < 1), if necessary also for partners of test persons or complete abstinence of intercourse (during study participation and follow up time and also for patients who receive PLX-PAD cells and withdraw early)
9. Signed written informed consent to participate in the study by subject.
10. No participation in other clinical trials 30 days prior to study enrolment

### Exclusion Criteria:

1. Uncontrolled hypertension (defined as diastolic blood pressure > 110 mmHg or systolic blood pressure > 180 mmHg during screening).
2. Poorly controlled diabetes mellitus (HbA1c > 9%)
3. Wounds with severity greater than Grade 2 on the Wagner Scale
4. Previous amputation of the talus, or above in the target limb.
5. Life-threatening ventricular arrhythmia - except if an ICD is implanted - or unstable angina pectoris - characterized by increasingly frequent episodes with modest exertion or at rest, worsening severity, and prolonged
6. ST segment elevation myocardial infarction and/or TIA/CVA within six (6) months prior to enrollment. Patients with severe congestive heart failure (i.e. NYHA Stage IV)
7. Infection of the involved extremity(ies) manifest by fever, purulence and severe cellulites
8. Subject requiring uninterruptible anticoagulation or that cannot be stopped for 24 hours prior to

### Protocol Synopsis

investigational treatment.
9. Blood clotting disorder not caused by medication.
10. Subject has malignancy undergoing treatment including chemotherapy, radiotherapy or immunotherapy.
11. Diagnosis of end stage renal disease requiring dialysis.
12. Pregnant or breast-feeding women or women of childbearing potential not protected by a highly effective contraceptive method of birth control.
13. Known allergies to protein products (horse or bovine serum, or porcine trypsin) used in the cell production process.
14. Body Mass Index (BMI) of 40 Kg/m2 or greater.
15. HIV, syphilis, positive at time of screening.
16. Active Hepatitis B, or Hepatitis C infection at the time of screening.
17. Positive for Tuberculosis but not LTBI (Latent Tuberculosis Infection). Patients with a weak TB-specific T cell response can be included in the trial only after exclusion of active tuberculosis and if they consent either to comply with a TB prophylactic therapy of Isoniazide (6-9 month) or avcombination therapy Isoniazide/Rifampicin
18. (3 month) or to undergo continuous monitoring of Tb symptoms
19. Subject undergoing hyperbaric oxygen treatment within four (4) weeks of inclusion and/or required throughout the trial.
20. Concomitant wound treatments that include growth factors or tissue engineered products, except for patients on chronic Becaplermin (topical), in which case, use of Becaplermin would have to cease for duration of study.
21. In the opinion of the investigator, the patient is unsuitable for cellular therapy.
22. Subject receiving systemic or direct target limb injection of antiangiogenic drugs.
23. Subject is currently enrolled in, or has not yet completed a period of at least 30 days since ending other investigational device or drug trial(s).
24. Subjects unwilling or unable to comply with the study procedures.
25. Subjects unwilling to consent to saving and propagation of pseudonymized medical data for study reasons
26. Subjects who are legally detained in an official institute

### Data and Safety Monitoring Board

The study will be monitored by an independent Data Safety and Monitoring Board (DSMB) that was specifically chosen to include an expert in Cardiology, in biostatistics and in vascular disease to ensure utmost competence and vigilance. The DSMB will meet before the start of the trial, then every month (following enrolment of first three subjects) in order to review the trial's progress, safety data (SAEs) and adherence to protocol.

### General Considerations

The primary objective is to evaluate the safety of PLX-C therapy The analysis dataset will include all patients having treatment using the PLX-C therapy and having been evaluated up to the termination visit (12 and 24 months, base on study design) visit (Per Protocol Set or Evaluable Patients). An additional analysis will be performed including all patients receiving injection with PLX-C and have completed 3 months of follow-up (Full Analysis Set or Intent-to-treat). Intent to treat analysis will be performed using the last measurement carried forward in the event of missing values. All patients having the treatment will be encouraged to stay in the study for the full study period. Patients will be allowed and encouraged to return to the study even if they have missed various study visits.

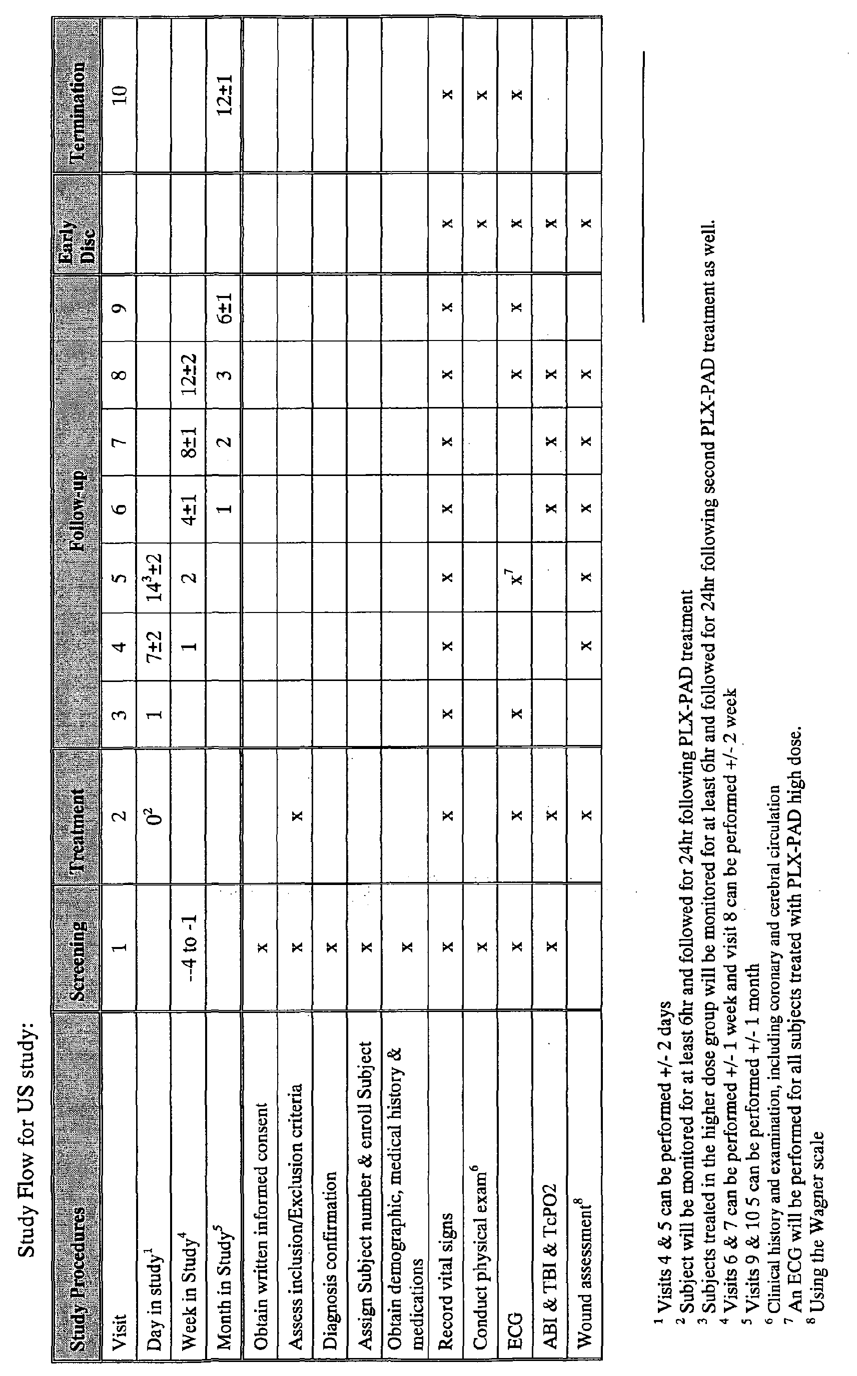

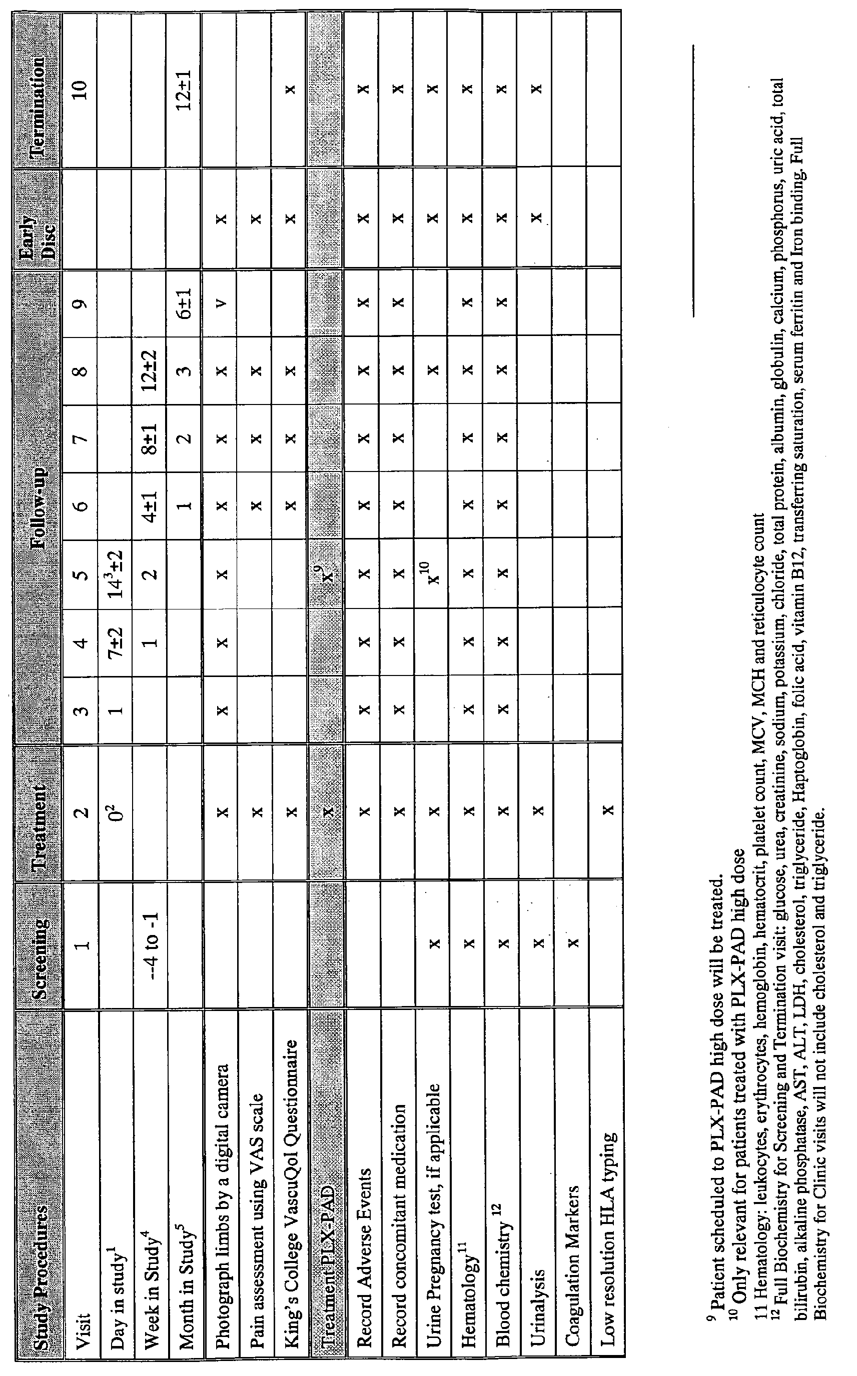

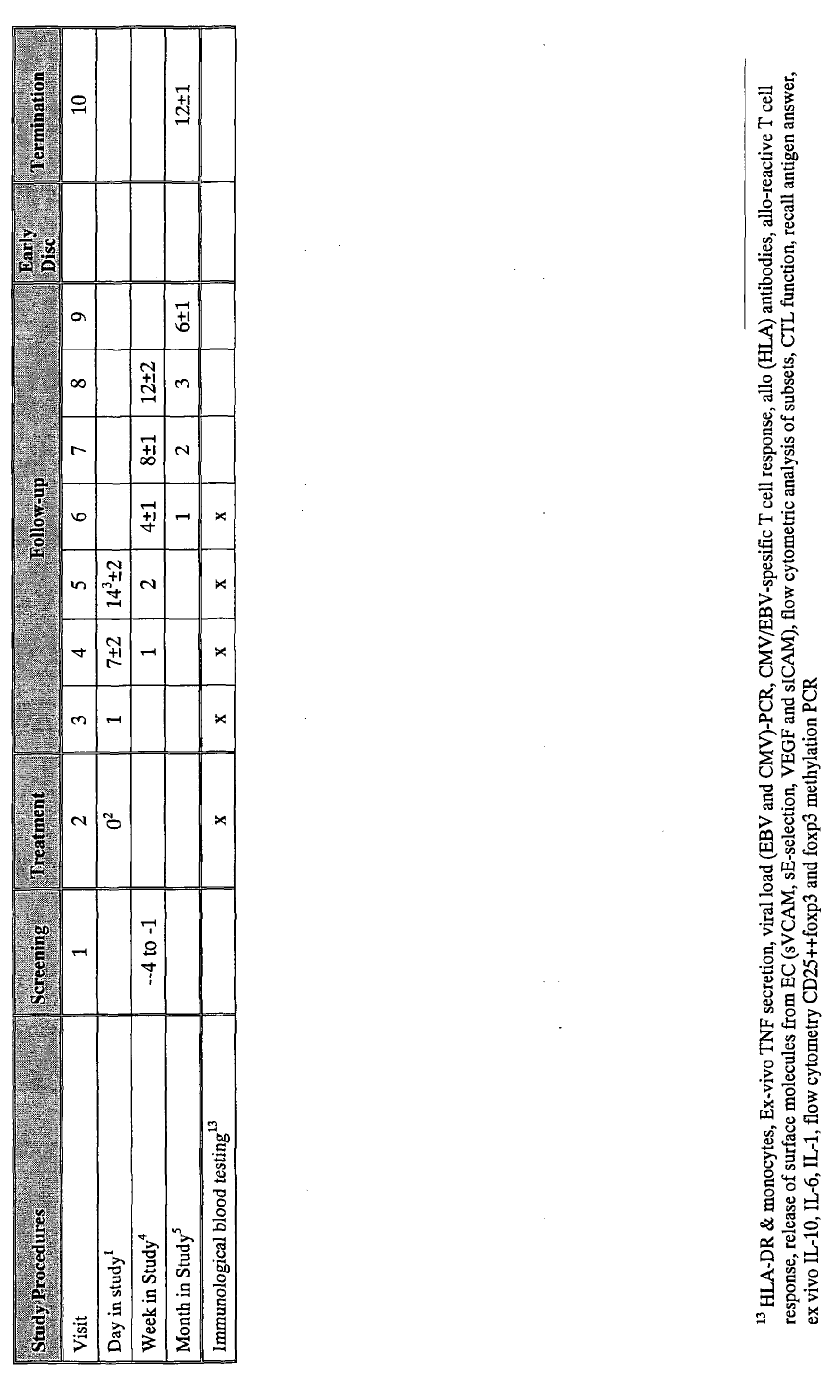

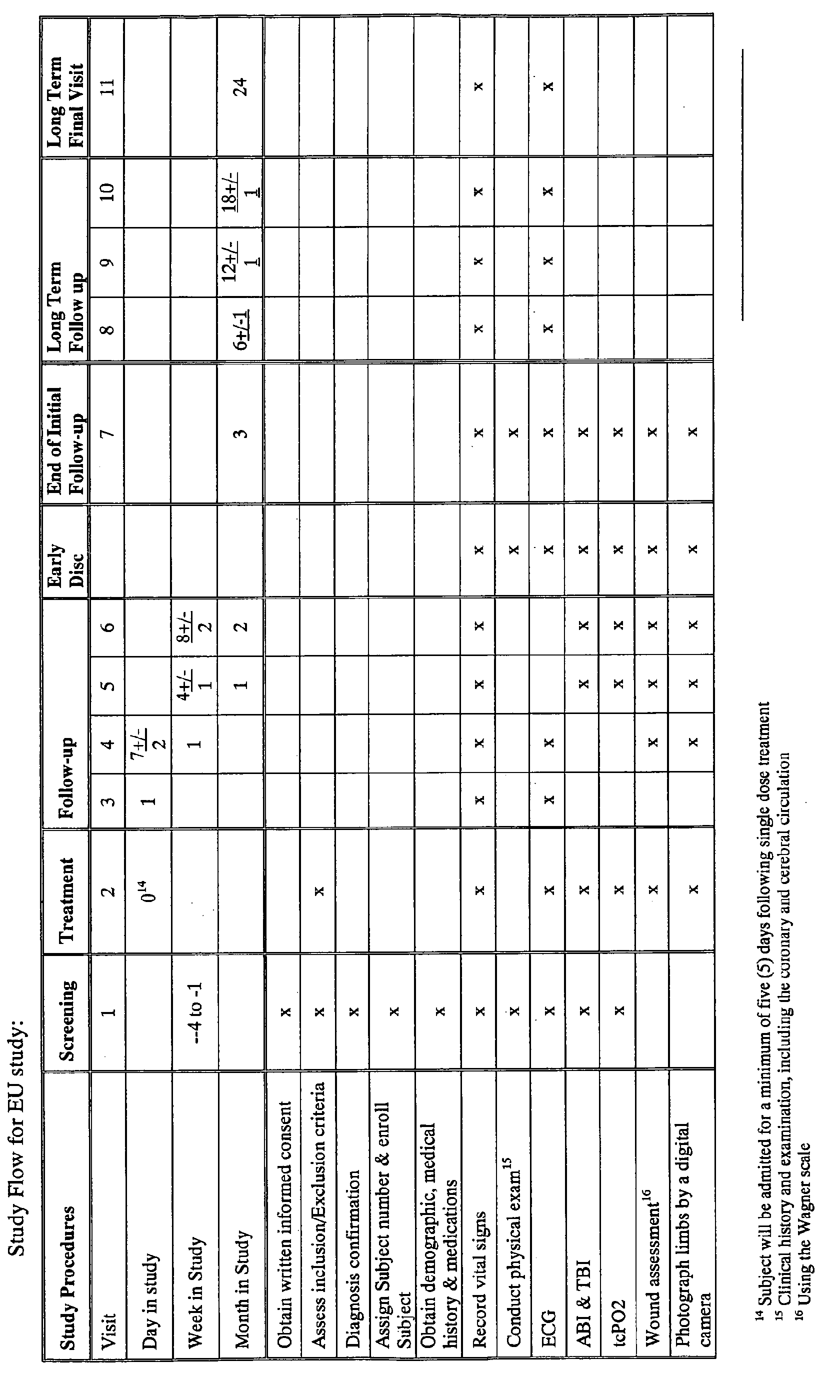

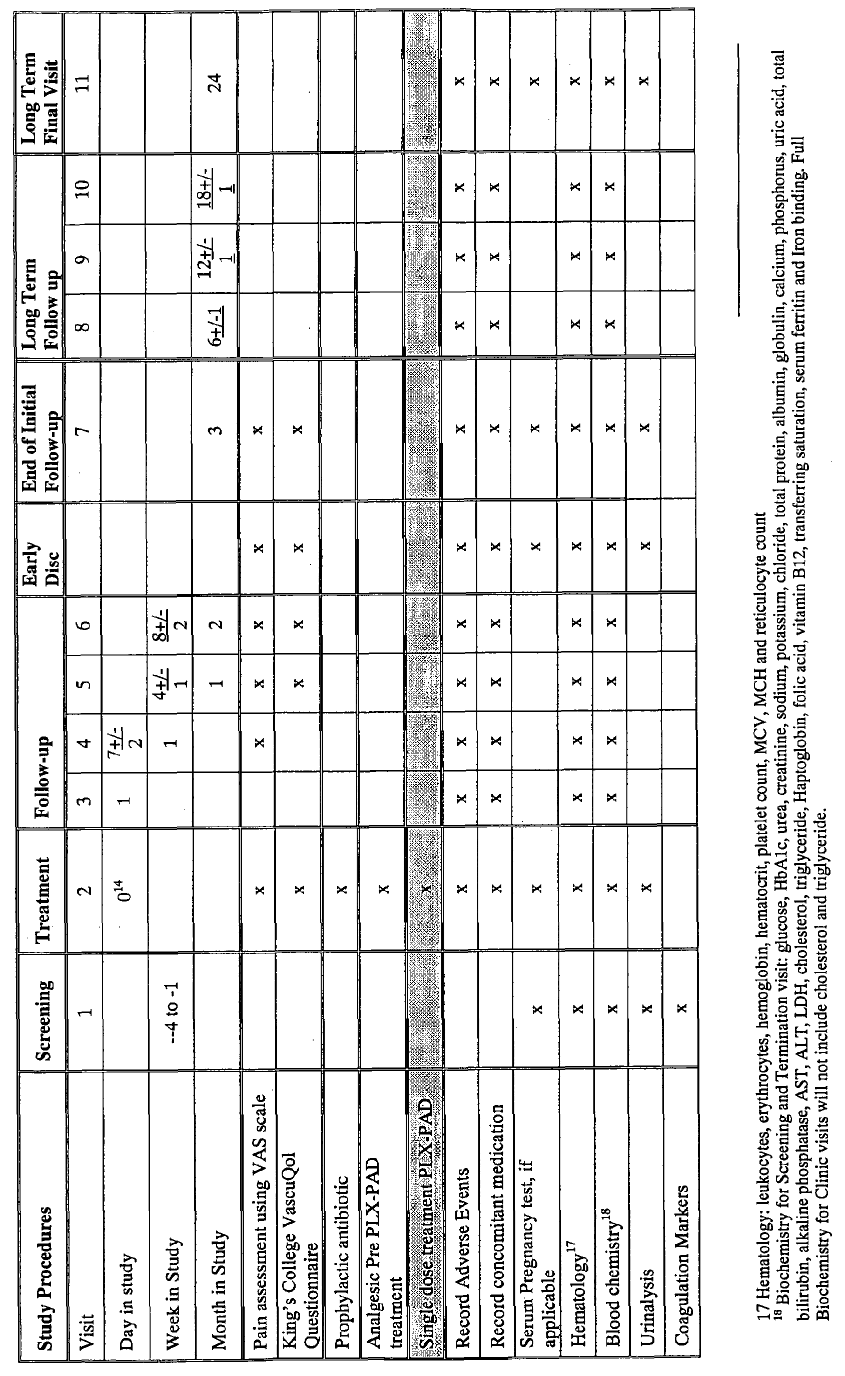

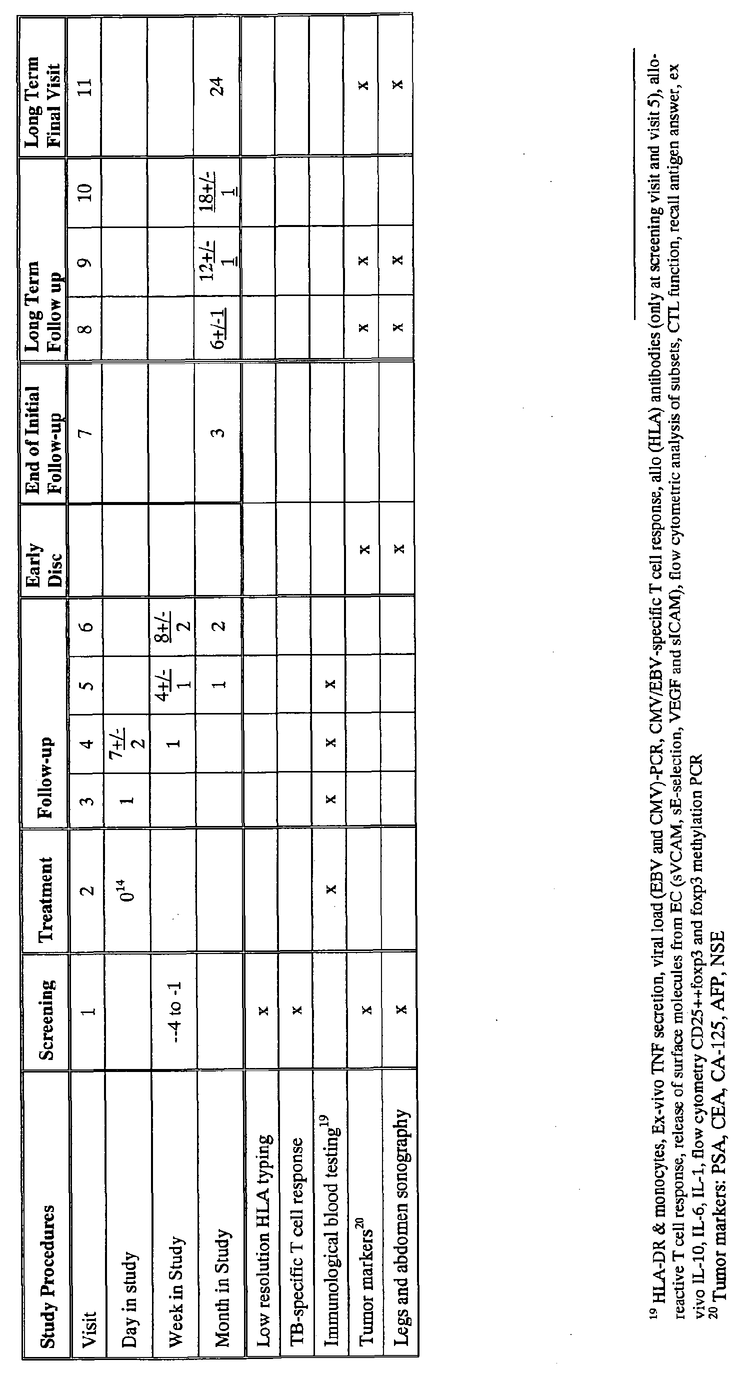

### Patient Disposition

A listing of all patients by disposition will be presented. Patient disposition will also be summarized by visit.

### Patient Characteristics

Baseline characteristics of gender, age, origin, body weight and BMI, will be summarized. Comparisons will be made using a simple two sample t-test for the continuous measurements and a Fisher's Exact test for the categorical measurements.

### Safety Analyses

Safety will be assessed by listing and summarizing treatment-emergent adverse events, and changes in laboratory analyses and vital signs. Treatment-emergent adverse events are events that first occurred or worsened after injection.

### Laboratory Tests

The Principal Investigator must assess the clinical significance of all abnormal laboratory values. All clinically significant abnormalities must be characterized by the Principal Investigator as treatment-related, not treatment-related, possibly treatment related, or of uncertain etiology. All abnormal laboratory values (outside the normal range for that site) judged treatment-related or of uncertain etiology must be repeated. Persistent abnormal values should be evaluated at the Principal Investigator's discretion.

### Quality Control and Quality Assurance

To ensure accurate, complete, and reliable data, the sponsor or its representatives will do the following:
1. Provide instructional material to the study sites, as appropriate.
2. Provide a start-up training session to instruct the investigator(s) and study coordinator(s). This session will give instruction on the protocol, the prompt and full completion of the clinical report forms, study procedures, and the transmission of data in a timely manner to Pluristem's clinical database for statistical analyses.
3. Make periodic visits to the study site.
4. Be available for consultation and stay in contact with the study site personnel by mail, telephone, and/or fax.
5. Review and evaluate case report form data and use.
6. Conduct quality review of database.

In addition, the sponsor or its representatives may periodically check a sample of the patient data recorded against source documents at the study site. The study may be audited by the sponsor and/or regulatory agencies at any time. Investigators will be given notice before an audit occurs.

To ensure the safety of participants in the study and to ensure accurate, complete, and reliable data, the investigator will keep records of laboratory tests, clinical notes, and patient medical records in the patient files as original source documents for the study. Investigator files will identify whether any clinical report form entries are source data. If requested, the investigator will provide the sponsor, applicable regulatory agencies, and/or applicable ethical review boards with direct access to original source documents.

The investigator has the responsibility of explaining the correct use of the investigational agent(s) to the patient and site personnel, ensuring that instructions are followed properly.

### Regulatory Considerations

This study shall be carried out in compliance with the PEI (Paul Erlich Institute), Germany and US FDA clinical trial regulations and according to the "Declaration of Helsinki."

### Injection and Delivery methods and rationale

Therapeutic angiogenesis is proposed to be effective for patients with infrainguinal arterial occlusive disease. Patients with critical limb ischemia most commonly have multi-segment infrainguinal disease, often involving the superficial femoral artery and either the popliteal or infrapopliteal arteries.

Collateral circulation most commonly involves branches of the profunda femoris artery. The rationale for thigh injection of angiogenic growth factors is to recruit additional perfusion from the profound femoris branches in the thigh. Since angiogenic growth factors function both by stimulating new blood vessel growth (angiogenesis) and by developing existing collateral vessels (arteriogenesis), it is reasonable to anticipate that therapeutic use of angiogenic agents will be more successful when profunda collaterals are recruited and/or developed. Injecting into the lower leg, close to the profoundly ischemic portion of the leg is considered necessary and the ischemic environment is one in which stimulated angiogenesis is most likely to occur.

Although the invention has been described in conjunction with specific embodiments thereof, it is evident that many alternatives, modifications and variations will be apparent to those skilled in the art.

In addition, citation or identification of any reference in this application shall not be construed as an admission that such reference is available as prior art to the present invention. To the extent that section headings are used, they should not be construed as necessarily limiting.

## Claims

1. A method of selecting a population of adherent cells of a placenta tissue suitable for treating ischemia, wherein said adherent cells are ex-vivo expanded, the method comprising:
(a) determining prior to transplantation in a candidate population of adherent cells of a placenta tissue the following parameters:
(i) percentage of viable cells in said candidate population;
(ii) immune phenotype of cells in said candidate population;
(iii) xeno-contamination in said candidate population;
(iv) sterility of said candidate population; and
(v) immunosuppressive activity of cells in said candidate population;
(b) selecting said candidate population according to the following values:
(i) at least 70% of viable cells in said candidate population; and
(ii) said immune phenotype comprising a positive marker expression of CD73, CD29 and CD105 and a negative marker expression of CD45, CD14 and HLA-DR of cells in said candidate population, wherein cells comprising said positive marker expression make up at least 90% of said candidate population and cells comprising said negative marker expression make up equally to or less than 3% of said candidate population;
(iii) no xeno-contamination in said candidate population;
(iv) sterility of said candidate population; and
(v) immunosuppressive activity of cells in said candidate population, as determined using a Mixed Lymphocyte Reaction (MLR) or a Phytohemagglutinin (PHA) assay,
thereby selecting a population of adherent cells of said placenta tissue suitable for treating ischemia.

2. The method of claim 1, wherein said population of adherent cells of a placenta tissue were produced by a method comprising culturing the adherent cells from the placenta tissue in a bioreactor under 3 dimensional (3D) culturing conditions which allow cell expansion; wherein said 3D culturing conditions comprise growing the cells in a culture medium.

3. The method of claims 1 or 2, wherein said xeno-contamination is selected from the group consisting of mycoplasma contamination and endotoxin contamination.

4. The method of claim 1, wherein said adherent cells are ex vivo expanded under 3D culturing conditions.

5. The method of claim 4, wherein said 3D culturing conditions are effected under perfusion.

6. The population of adherent cells of a placenta tissue having been selected to be suitable for transplantation according to any of the methods of claims 1-5 for use for treating ischemia in a subject in need thereof.

7. The population of adherent cells for the use according to claim 6, wherein the ischemia is peripheral artery disease (PAD).

## Patentansprüche

1. Verfahren zum Auswählen einer Population von adhärenten Zellen eines Plazentagewebes, die zum Behandeln von Ischämie geeignet ist, wobei die adhärenten Zellen ex vivo expandiert werden, wobei das Verfahren umfasst:
(a) Bestimmen der folgenden Parameter vor der Transplantation in einer Kandidaten-population von adhärenten Zellen eines Plazentagewebes:
(i) prozentualer Anteil lebensfähiger Zellen in der Kandidatenpopulation;
(ii) Immunphänotyp der Zellen in der Kandidatenpopulation;
(iii) Xenokontamination in der Kandidatenpopulation;
(iv) Sterilität der Kandidatenpopulation; und
(v) immunsuppressive Aktivität von Zellen in der Kandidatenpopulation;
(b) Auswählen der Kandidatenpopulation gemäß den folgenden Werten:
(i) mindestens 70 % lebensfähige Zellen in der Kandidatenpopulation; und
(ii) der Immunphänotyp weist eine positive Markerexpression von CD73, CD29 und CD105 und eine negative Markerexpression von CD45, CD14 und HLA-DR von Zellen in der Kandidatenpopulation auf, wobei Zellen, welche die positive Markerexpression aufweisen, mindestens 90 % der Kandidatenpopulation ausmachen und Zellen, welche die negative Markerexpression aufweisen, gleich oder weniger als 3 % der Kandidatenpopulation ausmachen;
(iii) keine Xenokontamination in der Kandidatenpopulation;
(iv) Sterilität der Kandidatenpopulation; und
(v) immunsuppressive Aktivität von Zellen in der Kandidatenpopulation, wie bestimmt unter Verwendung einer Mixed Lymphocyte Reaction (MLR) oder einer Phytohämagglutinin-(PHA)-Untersuchung,
wodurch eine Population von adhärenten Zellen des Plazentagewebes ausgewählt wird, die zum Behandeln von Ischämie geeignet ist.

2. Verfahren nach Anspruch 1, wobei die Population von adhärenten Zellen eines Plazentagewebes mittels eines Verfahrens, umfassend das Züchten der adhärenten Zellen aus dem Plazentagewebe in einem Bioreaktor unter dreidimensionalen (3D-) Zuchtbedingungen, die Zellexpansion ermöglichen, hergestellt wurde; wobei die 3D-Zuchtbedingungen das Züchten der Zellen in einem Kulturmedium umfassen.

3. Verfahren nach Anspruch 1 oder 2, wobei die Xenokontamination aus der Gruppe ausgewählt wird, die aus Mycoplasma-Kontamination und Endotoxin-Kontamination besteht.

4. Verfahren nach Anspruch 1, wobei die adhärenten Zellen unter 3D-Zuchtbedingungen ex vivo expandiert werden.

5. Verfahren nach Anspruch 4, wobei die 3D-Zuchtbedingungen unter Perfusion herbeigeführt werden.

6. Population von adhärenten Zellen eines Plazentagewebes, die nach einem der Verfahren der Ansprüche 1 - 5 ausgewählt wurde, um zur Transplantation geeignet zu sein, zur Benutzung zum Behandeln von Ischämie bei einem Subjekt, welches dies benötigt.

7. Population von adhärenten Zellen zur Verwendung nach Anspruch 6, wobei die Ischämie die periphere arterielle Verschlusskrankrankheit (PAD) ist.

## Revendications

1. Procédé de sélection d'une population de cellules adhérentes d'un tissu placentaire se prêtant au traitement de l'ischémie, dans lequel lesdites cellules adhérentes sont expansées ex vivo, le procédé comprenant :
(a) la détermination, avant la transplantation, dans une population candidate de cellules adhérentes d'un tissu placentaire, des paramètres suivants :
(i) le pourcentage de cellules viables dans ladite population candidate ;
(ii) le phénotype immunitaire des cellules dans ladite population candidate ;
(iii) la xénocontamination dans ladite population candidate ;
(iv) la stérilité de ladite population candidate ; et
(v) l'activité immunosuppressive des cellules dans ladite population candidate ;
(b) la sélection de ladite population candidate en fonction des valeurs suivantes :
(i) au moins 70 % de cellules viables dans ladite population candidate ; et
(ii) ledit phénotype immunitaire comprenant une expression de marqueur positive de CD73, CD29 et CD105 et une expression de marqueur négative de CD45, CD14 et HLA-DR de cellules dans ladite population candidate, dans lequel les cellules comprenant ladite expression de marqueur positive constituent au moins 90 % de ladite population candidate et les cellules comprenant ladite expression de marqueur négative constituent 3 % ou moins de ladite population candidate ;
(iii) pas de xénocontamination dans ladite population candidate ;
(iv) la stérilité de ladite population candidate ; et
(v) l'activité immunosuppressive des cellules dans ladite population candidate, telle que déterminée en utilisant une réaction lymphocytaire mixte (MLR) ou un essai de la phytohémagglutinine (PHA),
permettant ainsi de sélectionner une population de cellules adhérentes dudit tissu placentaire se prêtant au traitement de l'ischémie.

2. Procédé selon la revendication 1, dans lequel ladite population de cellules adhérentes d'un tissu placentaire a été produite par un procédé comprenant la culture des cellules adhérentes du tissu placentaire dans un bioréacteur dans des conditions de culture en 3 dimensions (3D) qui permettent l'expansion cellulaire ; dans lequel lesdites conditions de culture en 3D comprennent la culture des cellules dans un milieu de culture.

3. Procédé selon la revendication 1 ou 2, dans lequel ladite xénocontamination est sélectionnée dans le groupe constitué d'une contamination par mycoplasmes et d'une contamination par endotoxines.

4. Procédé selon la revendication 1, dans lequel lesdites cellules adhérentes sont expansées ex vivo dans des conditions de culture 3D.

5. Procédé selon la revendication 4, dans lequel lesdites conditions de culture en 3D sont réalisées sous perfusion.

6. Population de cellules adhérentes d'un tissu placentaire ayant été sélectionnée pour se prêter à la transplantation selon l'un quelconque des procédés selon les revendications 1 - 5 pour son utilisation dans le traitement de l'ischémie chez un sujet le nécessitant.

7. Population de cellules adhérentes pour son utilisation selon la revendication 6, dans laquelle l'ischémie est une maladie des artères périphériques (MAP).
